# EUROPÄISCHE PATENTSCHRIFT

(11) **EP 2 395 921 B1**
(45) Veröffentlichungstag und Bekanntmachung des Hinweises auf die Patenterteilung: **20.08.2014**
(21) Anmeldenummer: 10707448.6
(22) Anmeldetag: 16.02.2010
(51) Int. Cl.: A61B 10/00

(54) **VORRICHTUNG ZUM NACHWEIS VON ANALYTEN IN KÖRPERFLÜSSIGKEITEN**
DEVICE FOR VERIFYING ANALYTES IN BODILY FLUIDS
DISPOSITIF DESTINÉ À LA DÉTECTION D'ANALYTES DANS DES LIQUIDES ORGANIQUES

(30) Priorität: 16.02.2009 DE 102009010563
(43) Veröffentlichungstag der Anmeldung: 21.12.2011
(73) Patentinhaber: ulti med Products International GmbH, 22926 Ahrensburg (DE)
(72) Erfinder: ENGEL, Matthias W., 22926 Ahrensburg (DE); WILSON, Leslie, Sharon MA 02067 (US); JOHNSON, Paul, Blue Earth MN 56013 (US)
(74) Vertreter: Breit, Ulrich
(86) Internationale Anmeldenummer: PCT/EP2010/000948
(87) Internationale Veröffentlichungsnummer: WO 2010/091897

(56) Entgegenhaltungen:
- US-A1- 2003 190 259
- US-A1- 2006 018 800
- US-A1- 2006 127 274

## Beschreibung

Die vorliegende Erfindung betrifft eine Vorrichtung zum Bestimmen des Vorliegens und/oder der Menge eines oder mehrerer Analyten in einer Probe einer Körperflüssigkeit eines Menschen, die ein Behältnis zum Aufnehmen einer Körperflüssigkeitsprobe aufweist, mit einem Innenraum, der von einem Boden und einer Mantelfläche begrenzt wird, ferner zumindest einen Teststreifen mit einem saugfähigen Abschnitt sowie mit Reagenzien zum Bestimmen des Vorliegens und/oder der Menge eines oder mehrerer Analyten in der Probe, und ein Halteelement zur Aufnahme und zum Halten des oder der Teststreifen, wobei das Halteelement einer der umlaufenden Mantelfläche des Behältnisses entsprechende und angepasste Form aufweist.

Genauer gesagt, befasst sich die vorliegende Erfindung mit einer Vorrichtung zum Sammeln bzw. Testen einer flüssigen Probe, wie sie insbesondere bei Drogen- bzw. Vielfachdrogentests verwendet wird. Dazu wird aus einer Probe einer Körperflüssigkeit, bspw. Speichel oder Urin, das Vorhandensein bzw. die Menge eines oder mehrerer Analyten, insbesondere Missbrauchsdrogen bestimmt. Das Ergebnis kann dabei über Teststreifen visuell ermittelt werden.

Verschiedene analytische Vorrichtungen, mit welchen Analyten in einer Probe nachgewiesen werden können, sind bspw. aus der EP 0 225 054, der EP 0 183 442, EP 0 186 799, der EP 0 299 359, und der WO 89/06799 bekannt. All diese Vorrichtungen beschreiben den Einsatz von mit Reagenzien imprägnierten Test-Streifen in spezifischen Bindungsassays, bei welchen auf ein Ende des Test-Streifens eine flüssige Probe gegeben wird, die über bzw. durch den Teststreifen wandert. Dabei kann der Test-streifen in einer Hülle vorgesehen sein. Wenn in einer Probe der nachzuweisende Analyt vorliegt, kann er über ein in einer Nachweiszone immobilisiertes Bindungsreagenz bspw. über ein Sandwich-Assay oder über ein kompetitives Assay nachgewiesen werden.

Bekannt sind ferner Testkarten, die Teststreifen aufweisen, mit welchen parallel Amphetamine, sowie Drogen wie Extacy, Kokain, Crack oder Opiate und Cannabis nachgewiesen werden sollen.

Geeignete Nachweisverfahren sind beispielsweise Immunoassays, die hochsensitive Bestimmungsverfahren darstellen, die auf der Spezifität immunologischer Reaktionen beruhen. Der Nachweis der Substanzen erfolgt über eine gut nachweisbare und messbare Indikatorsubstanz, wie beispielsweise radioaktive Isotope, Enzyme, Farbstoffe, wie bspw. Gold-Sole, und Fluoreszenzfarbstoffe.

Bei den gegenwärtig vertriebenen Tests handelt es sich um Festphasentests. Ein markiertes Antigen oder ein markierter Antikörper, welcher spezifisch ist für die nachzuweisende Substanz, befindet sich dabei in einer ersten Zone, die zuerst in Kontakt kommt mit der flüssigen Probe. Befindet sich ein Analyt in der Probe, der von den spezifischen Antikörpern bzw. Antigenen erkannt wird, binden diese an den Analyten und wandern mit diesem durch den festen Träger bis zu einer zweiten Zone, in welcher ein weiteres Reagenz gebunden ist, das entweder den Analyten oder aber Antikörper/das Antigen ebenfalls spezifisch erkennt. Durch die Bindung des Analyten-Antikörper-/Analyten-Antigen-Reaktionsproduktes in der zweiten Zone kann der Analyt beispielsweise nachgewiesen werden durch eine Akkumulation der Markierungen, oder durch eine durch das Binden ausgelöste chemische Reaktion.

Festphasenimmunoassays in Kompetitiv- oder Sandwichtechnik sind dem Fachmann bekannt und brauchen daher nicht im Einzelnen erläutert zu werden.

Ferner ist aus der DE 200 21 659 U1 eine Vorrichtung bekannt, welche das Testen von Körperflüssigkeitsproben, insbesondere Urin, auf mehrere Missbrauchsdrogen zur selben Zeit vorsieht. Dabei werden auf einer Testkarte Teststreifen angeordnet, wobei jeder Streifen auf eine bestimmte Missbrauchsdroge empfindlich ist und einen visuellen Grenzwert hat, um das Vorhandensein oder die Abwesenheit einer bestimmten Droge anzuzeigen. Der dabei verwendete Behälter weist an seinem oberen Ende einen Verschlussdeckel auf, welcher einen Schlitz von solcher Größe hat, um eine Testkarte mit Teststreifen passend aufzunehmen. Die Testkarte ist durch den Schlitz derart einsetzbar, dass das eine Ende in die Urinprobe mit einer vorbestimmten Tiefe eingetaucht ist, wobei das visuelle Resultat jedes Teststreifens durch die Transparenz der Wandung des Behälters ohne Entfernung der Testkarte aus dem Behälter gesehen werden kann. Bei "positiv" getesteten Proben ist es dann notwendig, den geschlitzten Deckel durch einen zweiten durchgehenden Deckel auszutauschen und die Testkarte aus dem Behälter zu entfernen, um den Behälter dann verschlossen an ein zertifiziertes Labor einsenden zu können.

Die US 2003/190259 A1 offenbart eine Testvorrichtung, die einen Behälter mit mehreren Kammern, sowie Teststreifen aufweist. Ferner umfasst sie eine Stäbchen mit einer Schwammspitze, mit welcher eine flüssige Probe aufgenommen werden kann. Dieses kann dann in das Behältnis eingeführt werden und die Schwammspitze im Behältnis ausgedrückt werden, so dass die Probe in jeder der Kammern fließen kann. Der Oberbegriff von Anspruch 1 basiert auf dieses Dokument.

Ferner ist aus der US 2006/127274 A1 eine Testvorrichtung für flüssige Proben bekannt, bekannt, die ein durchsichtiges Behältnis umfasst, sowie ferner ein gekrümmtes Halteelement, das der runden Form des Behältnisses angepasst ist. Die Teststreifen sind dabei in dem Halteelement aufgenommen und durch die Behältniswand sichtbar.

Bei den im Stand der Technik bekannten Tests ist weiterhin nachteilig, dass für einen ersten Nachweis der Drogen stets ausreichende, d.h., verhältnismäßig große Mengen an Körperflüssigkeit vonnöten sind, um die Tests durchführen zu können. Insbesondere für den Nachweis von Drogen im Speichel werden aufgrund dessen Konsistenz und Verfügbarkeit größere Mengen benötigt, was den schnellen Drogennachweis, wie er bspw. bei direkten spontanen Kontrollen durchgeführt werden soll, im Speichel schwer macht. Daher wird bei den gegenwärtig verfügbaren Tests meistens auf Urinproben zurückgegriffen.

Vor dem obigen Hintergrund liegt der Erfindung die Aufgabe zugrunde, eine verbesserte bzw. einfacher ausgestaltete Vorrichtung zur Analyse von flüssigen Proben zu realisieren, welche keine zusätzlichen Hilfsmittel zur Vorbereitung der Probe benötigt.

Die Aufgabe wird ausgehend von einer eingangs genannten Vorrichtung dadurch gelöst, dass die Vorrichtung ferner ein längliches Probenentnahmeelement aufweist, das an einem seiner Enden einen saugfähigen, die Körperflüssigkeitsprobe aufnehmenden Probenaufnehmer aufweist, welches Probenentnahmeelement in das Behältnis zur Überführung der Probe in das Behältnis zumindest mit seinem den Probenaufnehmer aufweisenden Ende einführbar ist, und dass der Boden des Behältnisses eine zentrale Erhebung aufweist, über welche die Probe vom Probenaufnehmer durch dessen Auspressen auf der Erhebung dem zumindest einen Teststreifen zuführbar ist; ferner weist das Probenentnahmeelement und/oder der Probenaufnehmer Mittel auf zum Anzeigen des Vorliegens einer Menge der flüssigen Probe, die zum Bestimmen des Vorliegens und/oder der Menge eines oder mehrerer Analyten in der Probe ausreichend ist.

Mittels des länglichen Probenentnahmeelements sowie dem daran vorgesehenen saugfähigen Probenaufnehmer ist es in Zusammenwirkung mit der auf dem Boden des Behältnisses vorgesehenen Erhebung möglich, die in den saugfähigen Probenaufnehmer aufgesogene Körperflüssigkeitsprobe, bspw. Speichel, im Behältnis auszudrücken und den saugfähigen Abschnitten der im Behältnis vorliegenden Teststreifen einfach zuzuführen; dabei wird die aufgenommene Probe fast gänzlich verwertet, so dass vergleichsweise nur geringe Mengen an der Probe notwendig sind.

Die Aufgabe wird mit dieser Vorrichtung somit vollkommen gelöst.

Mit dem länglichen Probenentnahmeelement, das an einem seinem Ende ein Probenaufnehmer aufweist, wird also bspw. aus dem Mund oder Rachenraum eines Menschen eine Speichelprobe entnommen, d.h. der saugfähige Probenaufnehmer ist so beschaffen, dass der Speichel vom Probenaufnehmer aufgesogen wird. Das Material, aus dem der Probenaufnehmer ist, kann dabei jedes saugfähige Material sein, vorzugsweise ein Material mit watte- oder schwammähnlichen Eigenschaften, durch dessen Ausdrücken oder Auspressen die hierin enthaltene Probe auch gut aus dem Material geführt werden kann, und das darüber hinaus die Zusammensetzung des Speichels nicht verändert. Hierbei liegt es im Ermessen und Können des Fachmannes ein für diese Zwecke geeignetes Material vorzusehen. Das Probeentnahmeelement mit dem mit bspw. Speichel vollgesogenen Probenaufnehmer wird dann in das Behältnis gegeben, wo es auf die zentrale, also mittig auf dem Boden des Behältnisses vorgesehene Erhebung gedrückt und die in dem Probenaufnehmer enthaltene flüssige Probe, also bspw. der Speichel, dadurch ausgedrückt wird. Über die Erhebung fließt die Probe dann zu dem Seitenrand am Boden des Behälters, wo sich wiederum der saugfähige Abschnitt des zumindest einen Teststreifens befindet, der die Probe über Kapillarkräfte aufnimmt. Die Probe wandert dann über/auf dem Teststreifen, wodurch ev. in der Probe vorhandene Analyten durch die oben beschriebene Art und Weise nachgewiesen werden können.

Mit der erfindungsgemäßen Vorrichtung wird daher ein einfach zu handhabendes, auch für zu testende Speichelproben geeignetes Testgerät bereitgestellt, mit dem in einer Probe nachzuweisende Analyten schnell nachgewiesen werden können. Durch die mittige Erhebung wird außerdem sichergestellt, dass die in dem Probenaufnehmer enthaltene Probe gleichmäßig ausgedrückt und gleichmäßig an den umlaufenden inneren Rand des Behältnisses geführt wird. Die dort vorliegenden saugfähigen Abschnitte des/der Teststreifen werden daher gleichmäßig mit der Probe versorgt.

Ein "längliches" Probenentnahmeelement bedeutet vorliegend jedes stab- oder stäbchenförmige Element, das dazu geeignet ist, von entweder der zu testenden Person oder von einem Dritten an dessen einen Ende gegriffen zu werden, um mit dem anderen Ende bspw. in den Mund-/ Rachenraum eingeführt zu werden, zur Entnahme einer Speichelprobe. Das erfindungsgemäße Probeentnahmeelement weist daher ein Ende auf, an dem der Probenaufnehmer vorgesehen ist, und ein Greifende, sowie einen dazwischen liegenden länglichen, stab-/stäbchenförmigen Abschnitt. Es versteht sich aber, dass die Probe nicht auf eine Speichelprobe beschränkt ist, da das Probeentnahmeelement bspw. auch in ein die flüssige Probe enthaltende anderes Gefäß getaucht werden kann, wodurch sich der Probenaufnehmer ebenfalls mit der Probe voll saugen kann. Die so aufgenommene Probe wird dann wie oben beschrieben in das Behältnis geführt, und der Test zum Bestimmen des Vorliegens oder Menge eines Analyten durch Auspressen der Probe aus dem Probenaufnehmer und das Inkontaktkommen der Teststreifen mit der Probe gestartet.

Erfindungsgemäß ist bei der Vorrichtung dabei ferner bei dem Probenentnahmeelement und/oder dem Probenaufnehmer zumindest ein Mittel vorgesehen, über welches angezeigt wird, dass und ob genügend flüssige Probe, insbesondere Speichel, aufgenommen wurde.

In einer bevorzugten Ausführungsform ist dabei vorgesehen, wenn das zumindest eine Mittel ein in dem Probenentnahmeelement vorliegender Anzeigestreifen aus einem saugfähigen Material ist, der zumindest eine Zone aufweist, die von dem Probenaufnehmer entfernt liegt und in der durch Farbveränderung des Materials angezeigt wird, wenn genügend flüssige Probe, insbesondere Speichel, vorliegt. Das saugfähige Material ist dabei vorzugsweise ein Cellulose-aufweisendes Material und ermöglicht, dass die flüssige Probe durch bzw. über den Anzeigestreifen wandern und in die Zone gelangen kann, in der die Farbveränderung beobachtet wird. Dabei kann die Farbveränderung des Materials in dessen Dunkelfärbung liegen, die durch die über das Material aufgesaugte und wandernder flüssige Probe hervorgerufen wird.

In einer anderen Ausführungsform kann die Farbveränderung durch eine Reaktion des Materials mit der flüssigen Probe nach Art eines pH-Wert-Indikatorpapiers erfolgen, wonach das Material zumindest in der Zone seine Farbe ändert oder eine Farbe annimmt, wenn das Material zuvor farblos war. Es versteht sich, dass der Anzeigestreifen entweder insgesamt eine Farbveränderung erfährt, wenn die flüssige Probe über bzw. durch den Anzeigestreifen wandert, oder aber nur in der Zone, anhand der das Vorliegen einer ausreichenden Menge von flüssiger Probe angezeigt wird. Bei der letzten Ausführungsform ist die Zone entsprechend imprägniert, bzw. weist eine andere Zusammensetzung auf als das übrige Material des Anzeigestreifens. Ferner kann vorgesehen sein, dass nur die Zone über ein Fenster beobachtbar ist, während der übrige Anzeigestreifen mit einem sicht-undurchlässigem Material bedeckt ist.

In einer anderen Ausführungsform kann am Probenentnahmeelement in einer bestimmten Entfernung vom Probenaufnehmer am stäbchenförmigen Abschnitt des Probenentnahmeelements eine Markierung, bspw. in Form eines Strichs oder einer Einkerbung, vorgesehen sein, die als Anzeige dafür dienen soll, wie weit die Front der flüssigen Probe über den in dem Probenentnahmeelement vorliegenden Anzeigestreifen laufen muss, damit das Vorliegen einer ausreichenden Menge an flüssiger Probe in dem Probenaufnehmer gewährleistet ist. Es versteht sich, dass bei dieser Ausführungsform der Anzeigestreifen insgesamt einsehbar ist.

Der Anzeigestreifen, über welchen angezeigt wird, wann genügend flüssige Probe vorliegt, kann dabei bspw. in einer - im Querschnitt - U-förmigen Ausnehmung in dem stäbchenförmigen Abschnitt des Probenentnahmeelements angebracht sein, und mit einem seiner Enden in den Probenaufnehmer, der ein schwammähnliches Material aufweist, hineinragen, bzw. in diesen aufgenommen sein. Dadurch kann die flüssige Probe, die vom Probenaufnehmer aufgenommen wird, auf den Anzeigestreifen gelangen und über Kapillarkräfte über den Anzeigestreifen wandern.

Ferner ist bei einer Weiterbildung der erfindungsgemäßen Vorrichtung bevorzugt, wenn die Erhebung eine halbkugelförmige Erhebung mit dem höchsten Punkt mittig im Boden des Behältnisses darstellt.

Diese Maßnahme hat den Vorteil, dass über die halbkugelförmige Erhebung die in dem Probenaufnehmer enthaltene Probe einfach ausgedrückt werden kann, wonach die Probe aufgrund der halbkugeligen Beschaffenheit der Erhebung an der Erhebung herunter fließt und zum Rand des Bodens bzw. zu der Stelle, wo der Boden an die Mantelfläche übergeht, gelangt. Hier befindet wird die Probe von dem saugfähigen Abschnitt des zumindest einen Teststreifens aufgenommen und der Nachweis des Analyten auf dem Teststreifen nimmt seinen Lauf.

Bei der erfindungsgemäßen Vorrichtung ist dabei der Abstand des äußeren, umlaufenden Randes der halbkugelförmigen Erhebung von dem äußeren, umlaufenden Rand des Bodens - also dort, wo der Boden auf die Mantelfläche trifft - so bemessen, dass in diesen Abstand der saugfähige Abschnitt des Teststreifens passt, der Abstand kann jedoch auch bspw. größer sein. Ferner kann die Fläche des Abstandes zum Rand des Bodens hin abfallen, bzw. abschüssig sein, so dass die Fließrichtung der Probe von der Erhebung hinunter zum Rand des Bodens weiter unterstützt wird.

In einer weiteren Ausführungsform ist bevorzugt, wenn die Erhebung leistenförmige Elemente aufweist, die mittig von der Erhebung zum Boden hin geführt sind zum Ableiten der Probe an den umlaufenden Rand des Bodens des Behältnisses.

"Leistenförmig" soll vorliegend jedes Element bedeuten, dass die Funktion einer Ableitung der Probe von der Erhebung unterstützt, insbesondere schmale, längliche, entlang der äußeren Oberfläche der Erhebung angepasste Elemente, nach Art von Rippen.

Diese Maßnahme hat den Vorteil, dass die flüssige Probe gezielt und noch besser über den umlaufenden Rand des Bodens verteilt wird, so dass dann, wenn mehrere Test-streifen umlaufend mit ihrem saugfähigen Abschnitt am Rand angeordnet sind, auch sämtliche Teststreifen mit einer bestimmten Probenmenge versorgt werden.

Bei einer weiteren Ausgestaltung der erfindungsgemäßen Vorrichtung ist vorgesehen, wenn der Boden des Behältnisses im Bereich und umlaufend an der Mantelfläche entlang eine Vertiefung aufweist, zur Aufnahme der von der Erhebung abfließenden Probe.

Diese Maßnahme hat den Vorteil, dass der Speichel, der über die Erhebung durch Ausdrücken des Probenaufnehmers an der Erhebung herunter fließt, in einer Art Rinne bzw. umlaufenden Kanal gesammelt und konzentriert wird, so dass die oder der Teststreifen, die mit ihren saugfähigen Abschnitt in diese Vertiefung bzw. Rinne oder Kanal angebracht sind, noch zuverlässiger und ausreichend mit der flüssigen Probe versorgt werden.

Ferner ist in einer weiteren Ausführungsform vorgesehen, wenn der zumindest eine Teststreifen mit seinem die Probe aufnehmenden saugfähigen Abschnitt der Form der Vertiefung angepasst ist.

Hier kann beispielsweise vorgesehen sein, dass der saugfähige Abschnitt des oder der Teststreifen keilförmig angeschrägt ist und mit dem zugespitzten Ende in die Vertiefung reinragend vorgesehen ist. Diese Maßnahme hat den Vorteil, dass die flüssige Probe noch konzentrierter zu dem saugfähigen Abschnitt heran transportiert werden kann und dadurch ein sicherer Testablauf gewährleistet wird.

In einer Weiterbildung der erfindungsgemäßen Vorrichtung ist vorgesehen, wenn das Probenentnahmeelement an seinem dem Ende mit dem Probenaufnehmer entgegengesetzten Ende ein Verschlussmittel zum Verschließen des Behältnisses nach Einbringen des Probenentnahmeelements in das Behältnis aufweist.

Durch den Verschluss wird sichergestellt, dass der Testablauf ungestört und unbeeinflusst verlaufen kann. Ferner wird dadurch auch die sichere Transportfähigkeit der Vorrichtung gewährleistet, um bspw. die Vorrichtung zur Einbringung in ein Ablesegerät oder Scanner vorzubereiten, ohne dass Gefahr besteht, dass die Probe aus dem Behältnis ausläuft. Vorteilhafterweise ist der Verschluss dabei gleichzeitig der Griff des Probenentnahmeelements, so dass dieses hieran gegriffen und sicher gehandhabt werden kann. Die Speichelprobe wird hierbei also durch Einführen des anderen Endes des Probenentnahmeelements, also desjenigen, das den Probenaufnehmer aufweist, in den Mund bzw. Rachenraum und durch Aufnahme/Aufsaugen des hierin befindlichen Speichels in den Probenaufnehmer entnommen, während das Probenentnahmeelement über das andere Ende mit dem Verschluss, der gleichzeitig als Griff verwendet werden kann, gehandhabt wird, und zwar entweder von der zu testenden Person selber oder aber von einer anderen Person.

Dabei ist insbesondere bevorzugt, wenn das Verschlussmittel ein Drück-, Clip-, Dreh-, oder Bajonettverschluss oder ein Dichtungsring ist, das jeweils mit dem Behältnis und/oder dem Halteelement und/oder einem Einführstück zusammenwirkt. Es versteht sich, dass das Verschlussmittel - je nach Verschlussart - ein- oder mehrstückig aufgebaut sein kann. Ferner versteht es sich, dass das Verschlussmittel des Probenentnahmeelements mit an den entsprechenden Stellen des Behältnisses vorgesehenen Mitteln zusammenwirken kann, um das Behältnis fest zu verschließen.

So kann das obere Ende des Behältnisses, also das Ende, das verschlossen werden soll, bspw. ein Gegengewinde aufweisen, wenn das Verschlussmittel des Probenentnahmeelements ein Gewindegangstück aufweist.

Dabei können alternativ oder zusätzlich Verschlussmittel Probenentnahmeelementende vorgesehen sein, das in das Behältnis eingeführt wird, bspw. an der Position an der der Probenaufnehmer an den stäbchenförmigen Abschnitt fixiert ist. Diese Verschluss- oder Fixiermittel am Probenentnahmeelement können dann mit entsprechenden Mitteln im Halteelement oder im Behältnis und/oder mit einem Einführstück zur Fixierung des Probenentnahmeelements zusammenwirken.

Es versteht sich ferner, dass neben dem Verschlussmittel zusätzlich ein Griffelement vorgesehen sein kann, mithilfe dessen, bspw. im Falle eines Drehverschlusses - das Probenentnahmeelement gegriffen und in das Behältnis geschraubt werden kann.

Insbesondere ist hierbei bevorzugt, wenn an dem Verschlussmittel Mittel vorgesehen sind, um nach Einbringung des Probenentnahmeelements in das Behältnis die Entfernung des Probenentnahmeelements aus dem Behältnis zu blockieren. Hierbei ist insbesondere bevorzugt, wenn es sich bei den Mitteln um Rastmittel handelt, über welche das Herausdrehen oder -ziehen des Probenentnahmeelements nach dessen Einbringung in das Behältnis verhindert wird.

Hier ist also vorgesehen, dass mit dem Verschluss und dem Verschließen des Behältnisses durch den Verschluss, also nach Einbringen der Probe, bspw. Speichelprobe, sichergestellt wird, dass der Inhalt des Behältnisses nicht verändert wird, insbesondere die Probe nicht nachträglich verändert oder verfälscht wird. Ferner wird dadurch die Möglichkeit gegeben, dass die Probe, die sich im Behältnis befindet auch für weitere Tests verwendet werden kann ("B-Probe"). Hierbei ist für die zuständige Behörde bzw. für die den Test ausführende Person sichergestellt, dass nur bei einem unversehrt verschlossenen Behältnis die Probe unverfälscht, also so, wie sie dem Probanden entnommen wurde, im Behältnis vorliegt. Diese kann dann unbedenklich und zuverlässig für weitere Tests eingesetzt werden:

Das Probenentnahmeelement kann also in einer bevorzugten Ausführungsform derart aufgebaut sein, dass ein Verschlussabschnitt, bspw. ein Drehverschlussabschnitt, mit einem hierzu vergleichsweise dünneren, länglichen, stäbchenförmigen Abschnitt verbunden ist, der wiederum mit seinem anderen Ende mit dem sauffähigen Probenaufnehmer verbunden ist. Die Länge des Probenentnahmeelements ist dabei so bemessen, dass bei einem Einbringen des Elements und Verschließen des Behältnisses sich der saugfähige Probenaufnehmer entweder in einem komprimierten oder in einem unkomprimierten Zustand befindet, das Behältnis aber insgesamt verschlossen wird. Im komprimierten Zustand muss der Anwender Druck auf den Probenaufnehmer ausüben, um das Behältnis zu verschließen, im unkomprimierten Zustand muss der Probenaufnehmer zur Durchführung des Tests erst ausgepresst werden, wonach anschließend das Behältnis ohne Druck auf den Probenaufnehmer verschlossen werden kann.

Insbesondere ist hierbei bevorzugt, wenn das Verschlussmittel ein durchstechbares, selbstabdichtendes Element aufweist, über welches das Innere des Behältnisses nach dessen Durchstechen auch nach Aufbringung des Verschlussmittels auf das Behältnis zugänglich ist.

Diese Maßnahme hat den Vorteil, dass gerade bei der Notwendigkeit der Durchführung weitere Tests mit der ursprünglich entnommenen Probe die Möglichkeit besteht, einfach und schnell Zugriff auf diese ursprüngliche Probe zu haben, und zwar indem bspw. eine Spritze oder Kanüle, bzw. ein entsprechend ähnlich und spitz zulaufend geformtes Entnahmemittel durch das folienartige Element durchgeführt wird, wobei dieses durchstochen wird, und mit der Spritze/Kanüle/Entnahmemittel Teile der - nicht verbrauchten - Probe für weitere Tests entnommen werden.

In einer Ausführungsform der erfindungsgemäßen Vorrichtung ist bevorzugt, wenn das Behältnis ein zylinderförmiges Behältnis mit einem kreisförmigen Boden ist.

In einer weiteren, anderen Ausführungsform ist bevorzugt, wenn das Behältnis ein quaderförmiges Behältnis mit einem rechteckigen Boden ist. Diese Ausführungsform hat den Vorteil, dass sie bspw. über einen Scanner ausgelesen werden kann, da die ebenen Seiten des Behältnisses ein gleichmäßiges Ablesen ermöglichen.

Unabhängig von der spezifischen Form des Bodens und der Mantelfläche ist dabei unter einer "an die Form der Mantelfläche" angepassten Form des Halteelements zu verstehen, dass die Form des Halteelements bzw. das Halteelement an die die Innenseite des Behältnisses bildende Mantelflächenform umlaufend derart angepasst ist, dass sie den gesamten umlaufenden inneren Umfang der Mantelfläche bedeckt. Dies bedeutet, dass bei einer Ausbildung der Mantelfläche als - hohler - Zylinder, insbesondere Kreis- oder Eckzylinder, auch das Halteelement als zylindrisch, insbesondere kreis- oder eckzylindrisch, ausgebildet ist. Dabei versteht es sich, dass das Halteelement nicht zwingend auch die gesamte Höhe des Zylinders bedeckt. Dementsprechend ist das Halteelement als hohler Zylinder ausgebildet, das der inneren Zylinderform der Mantelfläche angepasst ist.

Ferner ist es bevorzugt, dass das Halteelement zumindest teilweise an der Innenseite der Mantelfläche des Behältnisses anliegt. Das Anliegen des Halteelements an der Innenseite der Mantelfläche verhindert somit den direkten Kontakt der flüssigen Probe mit dem Teil der Teststreifen, welcher die Reagenzien beinhaltet. Dadurch kommt nur der Teil der Teststreifen, welcher keine Reagenzien beinhaltet, mit der flüssigen Probe direkt in Kontakt. Ein Teil der Probe wird dann durch Kapillarkräfte in Längsrichtung der Teststreifen in den Bereich der Reagenzien gezogen. Hierdurch wird die Kontamination der flüssigen Probe verhindert, weshalb die Probe ohne Ausbau der Teststreifen an ein Labor zur weiteren Untersuchung eingesendet werden kann.

Des Weiteren ist es bevorzugt, dass das Halteelement zumindest teilweise von der Innenseite der Mantelfläche des Behältnisses beabstandet ist. Die Beabstandung des Halteelements ist vorteilhaft, da damit das In-Kontakt-Treten der zu untersuchenden Probe mit den Teststreifen erleichtert wird.

Gemäß einer weiteren Ausgestaltung ist vorgesehen, dass das Halteelement vorgespannt geformt und in das Behältnis geklemmt, angeordnet ist. Die Vorspannung des Halteelements ermöglicht einen guten Klemmsitz innerhalb des Behältnisses und verhindert ein Verrutschen des Halteelements und der darin angeordneten Teststreifen. Dies ist wiederum zur Verhinderung der Kontamination der Probe durch die auf den Teststreifen angeordneten Reagenzien wichtig.

In einer weiteren bevorzugten Ausgestaltung ist das Halteelement mit dem Behältnis, insbesondere mit der Innenseite der Mantelfläche und/oder des Bodens, zumindest teilweise verklebt. Die Verklebung des Halteelements mit dem Behältnis kann, wie der oben genannte Klemmsitz, ein Verrutschen des Halteelements und damit die Kontamination der Probe verhindern.

Es versteht sich, dass diese Verklebung auch in Kombination mit dem Klemmsitz angewendet werden kann, so dass die Verbindung zwischen Halteelement und Behältnis zusätzlich verbessert wird.

Alternativ hierzu ist es möglich, das Halteelement durch eine auf dem Boden angeordnete Führung im Behältnis anzuordnen. Eine Führung auf dem Boden des Behältnisses bietet den Vorteil, dass es den Einbau des Halteelements in das Behältnis während der Produktion erleichtert und damit beschleunigt.

Ferner ist es bevorzugt, dass das Halteelement Ausnehmungen aufweist, in welchem jeweils Teststreifen angeordnet sind. Die Ausnehmungen ermöglichen das Einpassen der Teststreifen und verhindern ein Verrutschen der Teststreifen innerhalb des Halteelements, also in Längsrichtung der Teststreifen selber.

Zudem ist es hierbei bevorzugt, dass die Ausnehmungen durch Stege getrennt sind, welche zumindest teilweise an der Innenseite der Mantelfläche des Behältnisses anliegen. Die Trennung durch Stege zwischen den einzelnen Teststreifen unterbindet ein In-Kontakt-Treten der Reagenzien der verschiedenen Teststreifen. Zusätzlich können die Stege ein Eintreten der flüssigen Probe in den Bereich der Teststreifen verhindern, in denen die Reagenzien angeordnet sind, so dass die flüssige Probe nur an das untere freie Ende der Teststreifen gelangen kann, an dem keine Reagenzien vorhanden sind. Dies verhindert somit die Kontamination der Probe selbst und die Beeinflussung der einzelnen Teststreifen bzw. deren Reagenzien untereinander.

Bei der zuvor erwähnten Vorrichtung ist es ferner bevorzugt, wenn die Mantelfläche des Behältnisses zumindest teilweise im Bereich der Teststreifen einen durchsichtigen Bereich aufweist. Auf diese Weise kann gewährleistet werden, dass eine direkte optimale Sicht auf die Teststreifen gegeben ist und das Ergebnis ohne Herausnehmen der Halteeinheit abgelesen werden kann.

In einer weiteren Ausführungsform ist bevorzugt, wenn das Halteelement - im Vergleich zu den Teststreifen - dunkler eingefärbt ist.

Diese Maßnahme hat den Vorteil, dass dadurch das Ablesen des Testergebnisses auf dem Teststreifen deutlich erleichtert wird, da der Kontrast zwischen dem dann helleren Teststreifen und dem Halteelement größer ist, als wenn das Halteelement die gleiche oder eine hellere Einfärbung aufweist als der zumindest eine Teststreifen.

Die zu untersuchende flüssige Probe kann dabei jede flüssige Probe einer Körperflüssigkeit eines Menschen - oder allgemein Säugetieres - sein, vorzugsweise Speichel.

Ferner ist bevorzugt, dass der durchsichtige Bereich von einem zumindest teilweise abnehmbaren Abdeckelement, insbesondere einer Sichtschutzfolie, verdeckt ist. Das Abdeckelement bietet den Vorteil, dass es das Ablesen der Testergebnisse durch den Probanden oder andere Unbefugte verhindert.

Ferner ist es hierbei bevorzugt, dass die Größe des Abdeckelements so gewählt ist, dass der an den Boden angrenzende Bereich der Mantelfläche von dem Abdeckelement nicht verdeckt ist. Durch die am unteren Ende der Mantelfläche freibleibende Fläche kann der Prüfer direkt erkennen, ob in dem Behältnis eine genügend große Menge der zu testenden flüssigen Probe enthalten ist, um das In-Kontakt-Treten mit den Teststreifen zu ermöglichen.

Gemäß einer weiteren bevorzugten Ausgestaltung ist vorgesehen, dass ein Überdeck-element, insbesondere eine Schutzfolie, die Teststreifen, welche in den Ausnehmungen des Halteelements angeordnet sind, zumindest teilweise überdeckt. Die Abdeckung der Teststreifen ist insbesondere in dem Bereich, welcher die Reagenzien enthält, von Vorteil, da dadurch eine direkter Kontakt zwischen der flüssigen Probe und den auf den Teststreifen vorhandenen Reagenzien verhindert wird. Die flüssige Probe tritt somit wiederum nur am unteren Teil der Teststreifen, an dem keine Reagenzien vorhanden sind, mit den Teststreifen in Kontakt. Ein Teil der flüssigen Probe wird durch Kapillarkräfte dann in den Bereich der Reagenzien gezogen. Dadurch wird wiederum die Kontamination der Probe verhindert.

Die erfindungsgemäße Vorrichtung kann zwischen 1 und 12 Teststreifen, insbesondere, 1, 2, 3, 4, 5, 6, 7, 8, 9, 10, 11 oder 12 Teststreifen, aufweisen, die je nach Einsatz in das Halteelement eingesetzt werden können. Insbesondere ist bevorzugt, wenn die Vorrichtung mindestens sechs Teststreifen aufweist.

In einer weitere Ausführungsform ist bevorzugt, wenn zusätzlich ein Einführstück vorgesehen ist, über welches das Probenentnahmeelement in das Behältnis einführbar ist. Das Einführstück weist vorzugsweise eine hohle, im wesentlichen zylindrische Form auf, mit einem oberen, dem Boden des Behältnisses entgegen gesetzten Ende, sowie einer unteren, dem Boden zugewandten Öffnung. Das Probenentnahmeelement kann in dieses Einführstück über dessen oberes Ende darin eingeführt werden, und reicht durch die untere Öffnung in das Behältnis hinein, und kann dadurch auf der am Boden vorgesehenen Erhebung ausgedrückt werden. Es versteht sich, dass das Einführstück entweder bereits in das Behältnis - und dadurch auch in das darin vorliegende Halteelement - eingeführt ist, und erst danach das Probenentnahmeelement eingeführt wird; andererseits kann vor Einführung in das Behältnis das Probenentnahmeelement außerhalb des Behältnisses bereits mit dem Einführstück zusammengeführt worden sein, und diese zusammengesetzte Anordnung in das Behältnis eingeführt wird.

Das Einführstück bietet den Vorteil, dass eine zusätzliche Fixierung des Probenentnahmeelements ermöglicht wird. Das Einführstück kann ferner in einer bevorzugten Ausführungsform am oberen Ende zwei seitliche Flansche aufweisen, die ein Einführen des Einführstücks durch Auflage der Flansche auf dem oberen Rand des Behältnisses begrenzen.

In einer weiteren Ausführungsform ist bevorzugt, wenn der Boden des Behältnisses Mittel aufweist, durch welche die Körperflüssigkeitsprobe von der Erhebung zu den Seitenrändern des Bodens, insbesondere den langen Seitenrändern bei einem rechteckigen Boden, zuleitbar ist. Dadurch wird gewährleistet, dass die Körperflüssigkeitsprobe von der Erhebung dem Bereich zugeführt wird, in den die saugfähigen Abschnitte der Teststreifen hineinragen, und so nach dem Ausdrücken ein Starten des Tests gesichert ist. In einer bevorzugten Ausführungsform sind dabei diese Mittel Erhebungen, die in einem Abstand von der Erhebung, auf der der Probenaufnehmer ausgedrückt wird, und jeweils angrenzend an die kurzen Seitenränder des Bodens vorgesehen sind.

Unabhängig von der jeweiligen Ausführungsform lässt sich die erfindungsgemäße Vorrichtung für jegliche Art von Drogen- bzw. Vielfachdrogentests verwenden. Dabei kann die vorliegende flüssige Probe gleichzeitig auf mehrere Missbrauchsdrogen getestet werden, darunter insbesondere Amphetamin, Barbiturate, Benzodiazepine, Kokain, Marihuana, Methadon, Methamphetamin, Methylenedioxymethamphetamin, Morphin, Opiate, Oxycodon, Phencyclidin, Propoxyphen, Trizyklische Antidepressiva, Buprenorphin, Cotinin, EDDP (2-Ethylidin-1,5-Dimethyl-3,3-Diphenylpyrrolidin) und Fentanyl.

Vorzugsweise wird für den Nachweis der Analyten, insbesondere von Drogen, ein Immunoassay eingesetzt, der auf dem Prinzip der kompetitiven Bindung beruht. Hierfür enthält der Teststreifen bspw. auf einer Testlinie eine mit Analyt-(insbesondere Drogen)-Protein-Konjugaten (bspw. reines Rinderserumalbumin) beschichtete Membran, bspw. eine Nitrocellulosemembran, sowie an einer Kontrolllinie einen polyklonalen Antikörper (Ziege) gegen Gold-Protein-Konjugat und ein Farbfeld mit Goldpartikeln, die mit spezifischen monoklonalem Antikörpern für die nachzuweisenden Drogen beschichtet und im Testfeld immobilisiert sind. Sind Drogen in der flüssigen Probe vorhanden, so konkurrieren diese mit ihren Konjugaten um Bindungsstellen auf ihrem spezifischen Antikörper. Während des Tests wandert die Probe, bspw. Urin oder Speichel, durch Kapillarkräfte "nach oben", d.h. von dem Probenaufnahmeende des Teststreifens zu dem anderen Ende des Teststreifens. Die Drogen-Protein-Konjugate auf dem Teststreifen gehen dabei in Lösung und wandern mit der Probe zu dem Testbereich des entsprechenden Drogennachweisstreifens. Drogen, die in Konzentrationen unterhalb der Nachweisgrenze in der flüssigen Probe, insbesondere Speichel, enthalten sind, sättigen die Bindungsstellen ihrer im Testfeld immobilisierten spezifischen Antikörper nicht ab, so dass die Antikörper in diesem Fall mit den Konjugaten reagieren, wodurch eine farbige Linie sichtbar wird. Ist die Konzentration der Drogen im Urin/der flüssigen Probe höher, so verdrängen die Drogenmoleküle aus dem Urin die Konjugate, bzw. besetzen alle Antikörper-Bindungsstellen, wodurch keine farbige Linie gebildet wird.

Zur Kontrolle, ob der Test richtig durchgeführt wurde, d.h. bspw., ob die Teststreifen richtig arbeiten, oder ob genügend Probe durch die Teststreifen aufgenommen wurde, können die Teststreifen einen Kontrollbereich aufweisen, in dem bei jeder Durchführung des Tests - unabhängig von ev. vorhandenen Drogen im Urin/der flüssigen Probe - eine farbige Linie auftaucht.

In einer bevorzugten Ausführungsform ist vorgesehen, wenn folgende Nachweisgrenzen für bestimmte Drogen vorgesehen sind: Amphetamin ab ca. 50 ng/ml und höher; Kokain: ab ca. 20 ng/ml und höher; Marihuana (Tetrahydrocannabinol): ab ca. 20 ng/ml und höher; Methamphetamin 50 ng/ml und höher; Methylendioxymethamphetamine: ab ca. 50 ng/ml und höher; Opiate: 40 ng/ml und höher; Phencyclidin: ab ca. 10 ng/ml und höher;.

Die Teststreifen können dabei einteilig oder mehrteilig ausgebildet sein, wobei "einteilig oder mehrteilig" vorliegend bedeutet, dass der Teststreifen aus einem Stück bzw. aus mehreren miteinander bspw. durch Überlappung verbundenen Teilen gebildet sein kann, welche aus unterschiedlichen oder gleichen Materialien mit unterschiedlichen oder gleichen Abmessungen gebildet sein können.

Es versteht sich ferner, dass einerseits ein oder mehrere, vorzugsweise zwei Analyten auf einem oder mehreren Teststreifen nachgewiesen werden kann, dass darüber hinaus auch Kontrollzonen vorgesehen sein können, anhand derer sich die Vollständigkeit und Korrektheit der Durchführung des Tests bestätigen lässt, und dass die Trägerstreifen Probenaufnehmer vorgeschaltet sein können, die bspw. der Aufnahme und Weiterleitung der Probe dienen. All diese Merkmale sind dem Fachmann bekannt und stellen übliche technische Merkmale dar, die dem allgemeinen Fachwissen zugerechnet werden. Diesbezüglich wird auch explizit Bezug genommen auf die Offenbarungen der bereits weiter oben erwähnten EP 0 225 054, der EP 0 183 442, EP 0 186 799, der EP 0 299 359 sowie der WO 89/06799, in welchen sowohl eine Reihe von nachzuweisenden Analyten als auch eine Reihe von Proben aufgeführt werden.

Ferner ist es gemäß einer weiteren Ausführungsform bevorzugt, dass die Teststreifen einen Alkoholtest umfassen. Der Alkoholtest ermöglicht es, die Probe nicht nur auf illegale Missbrauchsdrogen oder andere Analyten hin zu untersuchen, sondern dies auch mit dem Nachweis des Alkoholgehalts der Probe zu kombinieren. Insbesondere bei polizeilichen Untersuchungen kann diese Ausgestaltung somit schnell und ohne größeren Aufwand einen Hinweis auf eine eventuelle Fahruntüchtigkeit des Probanden geben.

In einer bevorzugten Ausführungsform erfolgt dabei ein Nachweis eines AlkoholGehalts von mindestens ca. 0,04 %. Dabei ist bevorzugt, wenn der Nachweis über eine Reaktion der Enzyme Alkoholoxidase, Peroxidase und einem Enzym-Substrat, wie bspw. Tetramethylbenzidin, in Gegenwart von Ethanol erfolgt.

Vorteilhafterweise wird mit dem Teststreifen zum Nachweis von Alkohol lediglich das Vorliegen von Alkohol an sich nachgewiesen. Es können jedoch weitere Maßnahmen getroffen werden, um auch die im den Probe vorliegende Menge an Alkohol zu bestimmen, bspw. über eine unterschiedliche, insbesondere eine mit steigendem Alkoholgehalt in der Intensität zunehmende Farbintensität der Reaktion, die anhand einer ebenfalls mitgelieferten Skala abgelesen werden kann.

Ferner ist in einer weiteren Ausführungsform bevorzugt, wenn die Vorrichtung zumindest einen Teststreifen zum Nachweis von Verfälschungen in der Probe aufweist.

Hierbei ist insbesondere bevorzugt, wenn die Teststreifen für die Verfälschungen eine Farbreaktion eingehen, die dann mit Farbfeldern einer Farbskala verglichen werden. Diese Farbskalen können der Vorrichtung beigefügt werden, so dass der Anwender eine Verfälschung direkt selber überprüfen/nachweisen kann. Dabei ist insbesondere bevorzugt, wenn die Farbreaktion mit "normal" (unverfälschte Probe) und in "abnormal" (verfälschte Probe) abgeglichen wird.

Ferner ist es bevorzugt, dass das Behältnis ein zylinderförmiges Behältnis mit einem kreisförmigen Boden ist und dass das Halteelement eine dem kreisförmigen Querschnitt der Mantelfläche angepasste gewölbte Form aufweist. Bei dem Behältnis handelt es sich somit um einen runden Becher, wodurch das Einpassen des Halteelements und später das Ablesen der Testergebnisse erleichtert wird. Das zylinderförmige Behältnis kann dabei vorzugsweise einen Durchmesser von ca. 1 cm bis ca. 15 ca, vorzugsweise von zwischen ca. 1 cm und 5 cm aufweisen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung der einzelnen Elemente einer Ausführungsform der erfindungsgemäßen Vorrichtung zum Testen von Flüssigkeitsproben im nicht zusammengebauten Zustand;
- Fig. 2a: eine schematische Darstellung der in Fig. 1 gezeigten Elemente im zusammen gebauten Zustand ohne Abdeckung;
- Fig. 2b: eine Darstellung wie in Fig. 2a mit partieller Abdeckung der Teststreifen bzw. des Behältnissen;
- Fig. 3: eine vergrößerte perspektivische Ansicht einer Ausführungsform des Bodens des Behältnisses der in Fig. 1 und 2 gezeigten Ausführungsform;
- Fig. 4: eine Detaildarstellung des den Boden aufweisenden unteren Abschnitts des Behältnisses der in Fig. 3 gezeigten Ausführungsform, mit dem Probenentnahmeelement in das Behältnis eingeführt und mit dem Probenaufnehmer auf der Erhebung im komprimierten Zustand;
- Fig. 5: eine Detaildarstellung des oberen, den Griff und das Verschlussmittel aufweisenden Abschnittes einer Ausführungsform des Probenentnahmeelements;
- Fig. 6: eine Detailansicht einer Ausführungsform des Verschlussmittels, in Zusammenwirkung mit entsprechend im Behältnis vorgesehenen Mitteln zum Verschließen der Vorrichtung;
- Fig. 7: eine detailliertere Draufsicht auf das Probenentnahmeelement einer Ausführungsform der erfindungsgemäßen Vorrichtung mit Mittel zum Anzeigen einer ausreichenden Menge an flüssiger Probe im Probenaufnehmer.
- Fig. 8: eine weitere Ausführungsform eines Probenentnahmeelements, im Längsschnitt (A) und in Explosionsdarstellung (B);
- Fig. 9: eine weitere Ausführungsform der erfindungsgemäßen Vorrichtung, wobei in (A) die teilweise zusammengesetzte Form gezeigt ist; in (B) das aus dem Behältnis herausgenommenen Halteelement; in (C) das Probenentnahmeelement und in (D) ein Einführstück für das Probenentnahmeelement;
- Fig. 10: einen Querschnitt durch die in Fig. 9 gezeigte zusammengesetzte Ausführungsform; und
- Fig. 11: eine Draufsicht auf den Boden des Behältnisses der in Fig. 9 und 10 gezeigten Ausführungsform.

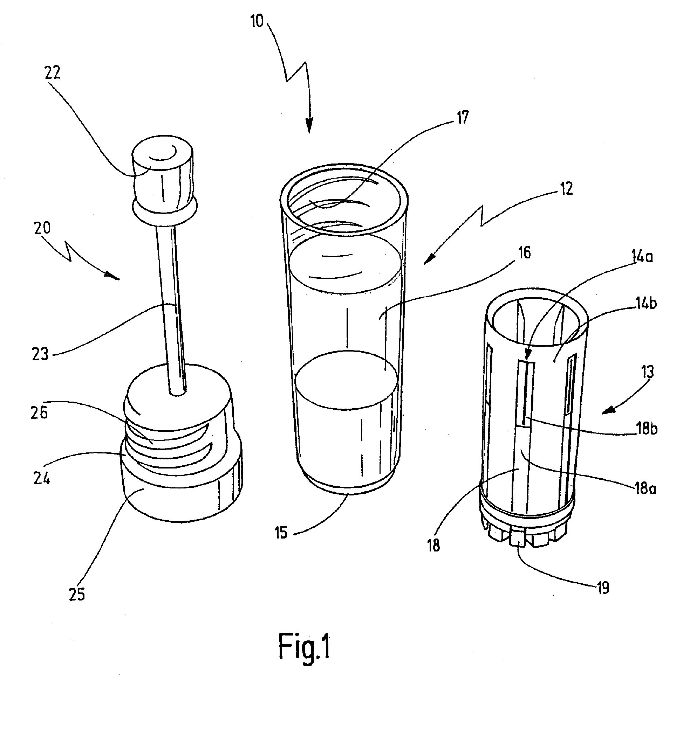

In Fig. 1 ist eine Ausführungsform einer erfindungsgemäßen Vorrichtung 10 zum Testen von Flüssigkeitsproben gezeigt, wobei die einzelnen Elemente der Vorrichtung 10 gezeigt sind, und sich die Vorrichtung 10 insgesamt im nicht zusammengebauten Zustand befindet.

Die Vorrichtung 10 umfasst ein Behältnis 12, ein in das Behältnis 12 anzuordnendes Halteelement 13 für einen oder, wie in Fig. 1 gezeigt, mehrere Teststreifen 18. Die Teststreifen 18 weisen saugfähige Abschnitte 19 auf, über die die flüssige Probe aufgenommen wird und dadurch der Test auf dem Teststreifen 18 gestartet wird.

Bei der in Fig. 1 dargestellten Ausführungsform ist das Behältnis 12 zylinderförmig mit einem kreisförmigen Boden 15 und einer rund gewölbten Mantelfläche 16 ausgebildet. Das Behältnis 12 weist des Weiteren an seinem oberen Ende ein Gewinde 17 auf, welches durch Zusammenwirken mit entsprechenden Gegenmitteln dem Verschluss des Behältnisses 12 dient.

Das Halteelement 13 weist längsaxiale Ausnehmungen 14a auf, welche durch zwischen den Ausnehmungen 14a liegende Stege 14b voneinander getrennt werden. Die Ausnehmungen 14a und die Stege 14b sind nach oben hin begrenzt durch eine freie Fläche des Halteelements 13. Die Ausnehmungen 14a des Halteelements 13 sind jedoch zum dessen unteren Ende hin durchgehend. In die Ausnehmungen 14a des Halteelements 13 sind in Fig. 1 Teststreifen 18 eingebracht. Die Teststreifen 18 sind in dieser Ausführungsform genau eingepasst in die Ausnehmungen 14 des Halteelements 13 und sind durch die Stege 15 voneinander getrennt. Der Teststreifen 18 teilt sich auf in einen Abschnitt 18a, in welchem die Testergebnisse abgelesen werden können, und einen Abschnitt 18b, auf welchem die Bezeichnung des jeweiligen zu testenden Analyten vermerkt ist. Der Abschnitt 18a weist somit die Reagenzien auf, welche durch Reaktion mit den zu testenden Analyten verantwortlich für das Anzeigen eines Testergebnisses sind. Durch die der Mantelfläche 16 angepasste Form des Halteelements 13, das sich in dem in Fig. 1 gezeigten Beispiel über den gesamten inneren Umfang der Mantelfläche 16 umlaufend erstreckt, lassen sich die Ergebnisse somit sehr einfach und ohne Beeinträchtigung der Sicht ablesen. Ferner weisen die Teststreifen 18 noch saugfähige Abschnitte 19 auf, über die die Probe auf den Teststreifen 18 aufgenommen wird.

In Fig. 1 ist ferner mit 20 das Probenentnahmeelement gezeigt. Dieses weist an seinem in das Behältnis 12 einzuführenden Ende einen Probenaufnehmer 22 auf, mit welchem bspw. aus dem Mund- bzw. Rachenraum eine Speichelprobe entnommen werden kann. Der Probenaufnehmer 22 ist dabei Watte- bzw. Schwammähnlich aufgebaut, bzw. aus einem saug- und Flüssigkeitsspeicherungsfähigem Material. Über einen länglichen, stäbchenförmigen Abschnitt 23 ist der Probenaufnehmer 22 mit dem Endabschnitt 24 verbunden, der in der in Fig. 1 gezeigten Ausführungsform gleichzeitig Griff 25 und Verschlussmittel 26 aufweist. Bei den in Fig. 1 gezeigten Verschlussmitteln 26 handelt es sich um Drehverschlussmittel, also um ein an dem Endabschnitt vorgesehenes Gewinde, das wiederum bei Einbringen des Probenaufnehmers 22 in das Behältnis 12 mit dem Gegengewinde 17 des Behältnisses 12 zusammenwirkt.

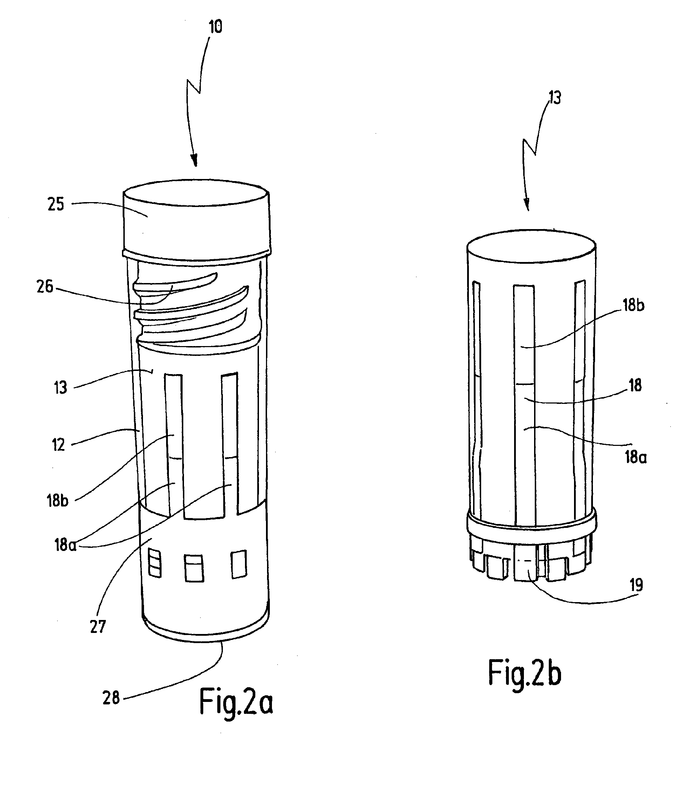

Fig. 2a zeigt die in Fig. 1 gezeigten Elemente einer Ausführungsform der erfindungsgemäßen Vorrichtung im zusammengesetzten Zustand. Fig. 2 ist dabei zu entnehmen, dass das Halteelement 13 in das Behältnis 12 eingebracht ist, so dass die saugfähigen Abschnitte 19 der sich im Halteelement 13 befindlichen Teststreifen 18 mit dem Boden 15 des Behältnisses 12 in Kontakt stehen. Ferner ist das Probenentnahmeelement 20 in das Behältnis 12 eingebracht, derart, dass der Probenaufnehmer 22 in das Behältnis 12 eingeführt ist, und das Verschlussmittel 26 mit dem Gegengewinde 17 des Behältnisses 12 zum Verschluss des Behältnisses 12 zusammenwirkt.

Fig. 2b zeigt die gleiche Ausführungsform wie in Fig. 2a, mit dem weiteren Merkmal eines partiellen Abdeckelements 27 der Teststreifen 18. Dieses Abdeckelement 27 befindet sich dabei, bspw. mittels Verkleben, auf der äußeren Oberfläche des Behältnisses 12, und zwar unterhalb des Bereiches 18a, also des Bereiches, auf dem die Testergebnisse angezeigt werden. Auf dem Abdeckelement 27 kann bspw. eine Anleitung zu finden sein, wie die Testergebnisse auszuwerten sind, d.h. bspw., dass bei Auftreten einer Bande im Bereich 18a des Teststreifens 18 der Proband positiv auf einen bestimmten Analyten getestet wurde ("pos"), und dass bei Auftreten von zwei Banden im Bereich 18a der Proband negativ ("neg") für den Analyten ist; ist keine Bande im Bereich 18a zu erkennen, ist der Test ungültig ("invalid").

Die Mantelfläche 16 des Behältnisses 12 kann dabei am unteren, an den Boden 15 angrenzenden Rand, einen Abschnitt 28 aufweisen, welche von dem Abdeckelement 27 nicht verdeckt sind. Dieser freibleibende Abschnitt 28 dient bspw. der Möglichkeit zur Kontrolle, ob die Menge der zu testenden flüssigen Probe ausreichend ist.

Das Halteelement 13 kann, wie bereits weiter oben erwähnt, im Vergleich zur Farbe der Teststreifen 18 dunkler eingefärbt sein, was das Ablesen des Testergebnisses im Bereich 18a erleichtert, da durch die dunklere Einfärbung der Kontrast zu dem Teststreifen größer ist als bei einem hell eingefärbten Halteelement 13.

Es versteht sich, dass das Behältnis 12 auch insgesamt auf seiner äußeren Oberfläche mit einem Abdeckelement versehen sein kann, und dass im Bereich 18a, also dort, wo das Testergebnis abgelesen wird, ein Bereich ist, in dem das Abdeckelement vom Behältnis abgelöst werden kann, so dass das Testergebnis und der Bereich 18a der Teststreifen von außen einsehbar ist. Dabei kann das Abdeckelement bspw. auf der äußeren Oberfläche mit dem Behältnis verklebt sein, wobei für den Bereich, der zum Testablesen abgelöst werden soll, bspw. eine leichtere, ablösbare Verklebung gewählt wird als für die auf der Oberfläche des Behältnisses verbleibenden Bereiche.

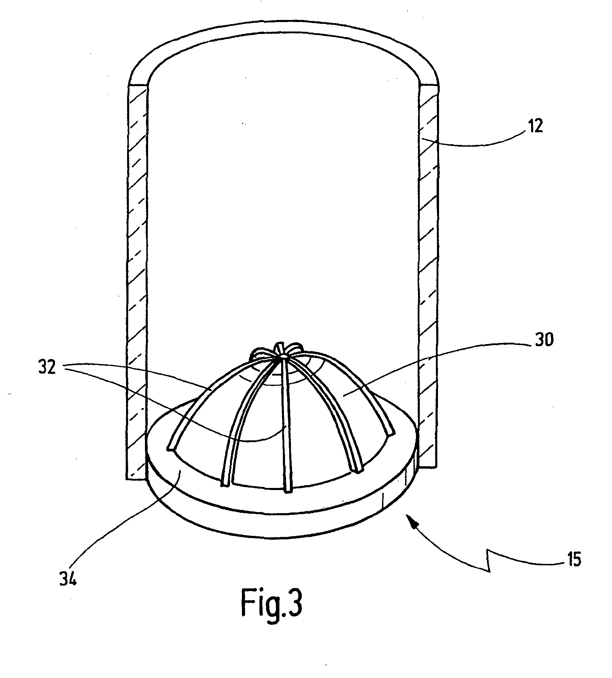

In Fig. 3 ist eine vergrößerte Darstellung der inneren Ausgestaltung des Bodens 15 des Behältnisses 12 gezeigt, wobei die Seitenwand des Behältnisses 12 zur Darstellung der Bodenausgestaltung aufgeschnitten ist. Fig. 3 ist zu entnehmen, dass der Boden 15 eine Erhebung 30 in Form einer aufgeschnittenen Halbkugel aufweist, wobei die Halbkugel mit der Wölbung in Richtung anderes Ende des Behältnisses 12 zeigend auf den Boden 15 mittig aufgesetzt ist. Die Erhebung 30 weist darüber hinaus Leistenförmige Elemente 32 auf, die ausgehend von dem höchsten Punkt der Erhebung 30 seitlich an dieser herabgeführt sind. Der Boden 15 weist darüber hinaus eine Vertiefung bzw. Probensammelrinne 34 auf, die umlaufend am Rand zur Mantelfläche gebildet ist, und die die Probe, die von der Erhebung 30 und durch die Leistenförmigen Elemente 32 von dieser heruntergeführt wird, aufnimmt. In diese Vertiefung/Probensammelrinne 34 sind die saugförmigen Abschnitte 19 der Teststreifen 18 aufgenommen, so dass die sich in der Probensammelrinne 34 befindliche Probe durch Kapillarkräfte über die saugfähigen Abschnitte 19 in die Teststreifen 18 aufgenommen werden und der Bestimmungstest für den oder die Analyten auf dem oder den Teststreifen 18 ablaufen kann.

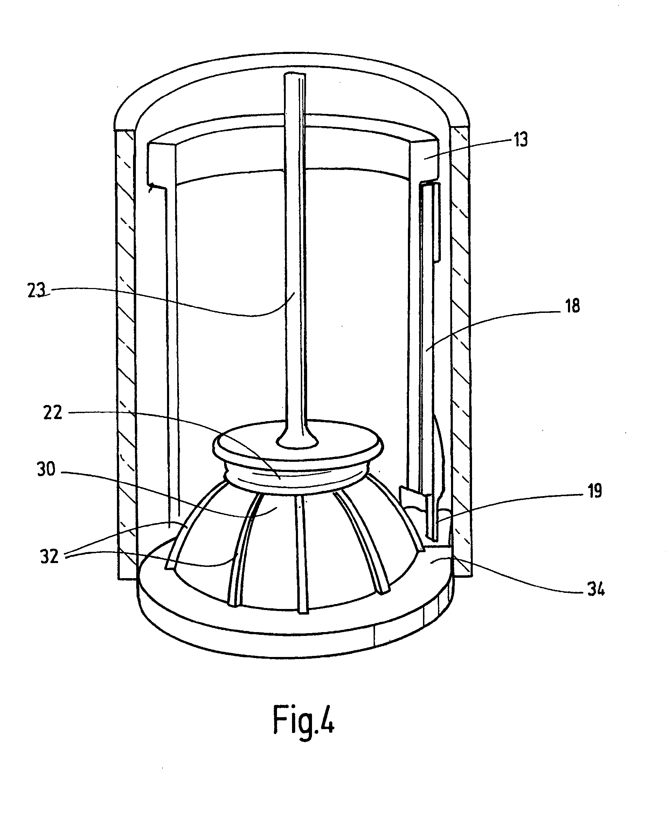

Fig. 4 zeigt eine Darstellung, in der das Probenentnahmeelement 20 mit seinem den Probenaufnehmer 22 aufweisenden Ende auf die Erhebung 30 des Bodens 15 des Behältnisses 12 aufgedrückt ist. Die in dem Probenaufnehmer 22 aufgenommene und darin gespeicherte Probe wird dadurch auf der Erhebung 30 ausgedrückt und läuft seitlich an dieser in die Probensammelrinne 34 hinunter. Der Ablauf der Probe von der Erhebung wird durch die an der Erhebung 30 vorgesehenen Leistenförmigen Elemente 32 zusätzlich gefördert, bzw. durch diese eine gleichmäßige Verteilung der Probe in die umlaufende Probensammelrinne 34 unterstützt.

Fig. 4 ist ferner zu entnehmen, dass der Teststreifen 18, der in das Halteelement 13 eingebracht ist, mit seinem saugfähigen Ende 19 in die Probensammelrinne 34 hineinragt.

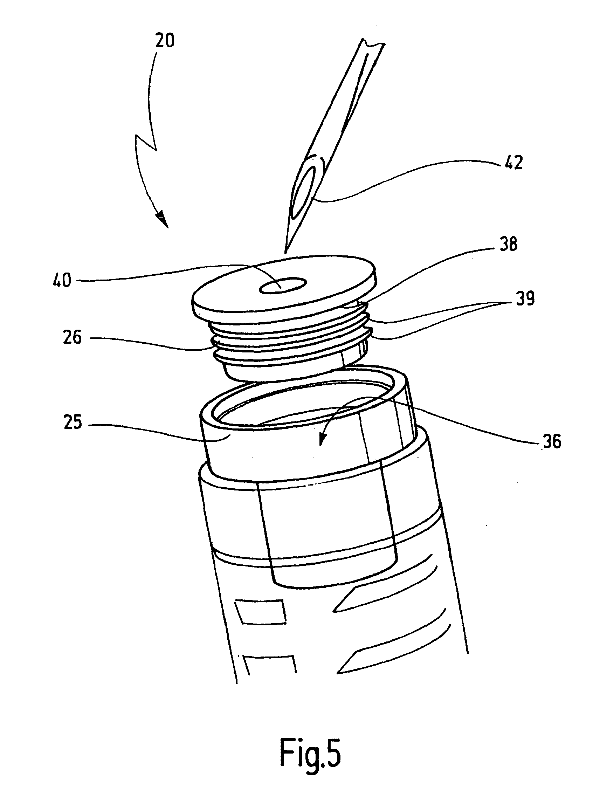

Fig. 5 zeigt detailliert eine Ausführungsform des Endes des Probenentnahmeelements 20, das den Griff 25 und/oder die Verschlussmittel 26 aufweist. Mittig im Kopf des Probenentnahmeelements 20 ist ein B-Probensammelbehälter 36 vorgesehen, mit einer fest zu verschließenden, nicht spurenfrei wieder zu öffnenden Verschlusskappe 38. Die Verschlusskappe 38 weist seitlich umlaufende Verschlusslamellen 39 sowie ein selbstabdichtendes, durchstechbares Probeentnahmemittel 40 auf, über welches bspw. mit einer Spritze oder Kanüle Material für die B-Probe entnommen werden kann, was durch die teilweise dargestellte Spritzenspitze 42 schematisch angedeutet ist.

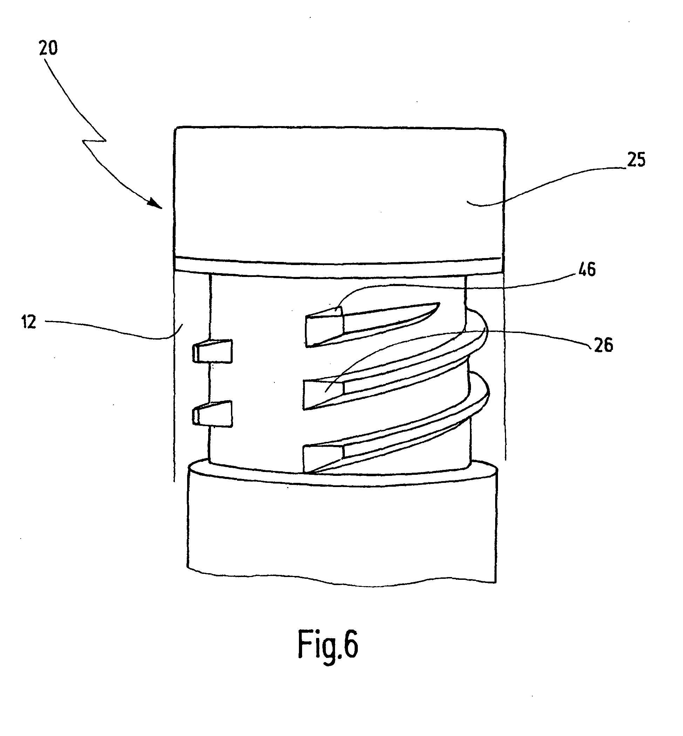

In Fig. 6 zeigt schließlich eine vergrößerte Darstellung des die Verschlussmittel 26 aufweisenden Endes des Probenentnahmeelements 20. Der äußerste Endabschnitt bildet eine Art Griff 25, und die Verschlussmittel 26 sind in Form eines Gewindes ausgebildet, das in ein am Behältnis 12 vorgesehenes Gegengewinde 17 verschließend eingreift. Das in Fig. 6 mit 44 bezeichnete Gewindegangstück weist eine Raste 46 auf, welche bei Betätigung eine Sicherung des Gegengewindes 17 ermöglicht. Die Raste 46 kann durch eine entsprechende Markierung bzw. durch einen entsprechenden Hinweis auf dem äußeren Behältnis besonders hervorgehoben werden, um auf die Möglichkeit der Unversehrtheitskontrolle hinzuweisen.

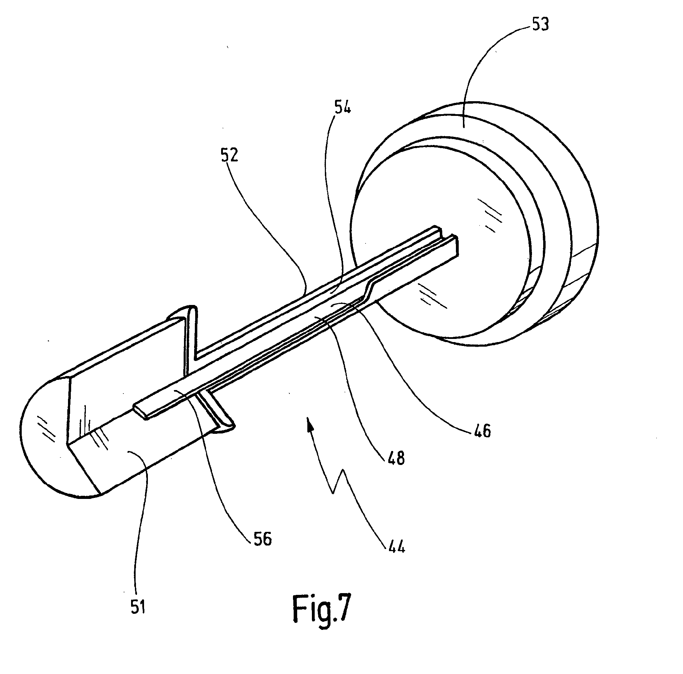

Fig. 7 zeigt eine detaillierte Ansicht einer weiteren Ausführungsform eines Probenentnahmeelements 44 der erfindungsgemäßen Vorrichtung 10. Das Probenentnahmeelement 44 weist dabei ebenfalls einen Probenaufnehmer 51, einen stäbchenförmigen Abschnitt 52 sowie einen Griffabschnitt 53 auf. Der stäbchenförmige Abschnitt 52 weist ferner eine - im Querschnitt gesehene - U-förmige Ausnehmung 54 auf, in die ein Anzeigestreifen 46 zum Anzeigen des Vorliegens einer für die Durchführung des Assays ausreichenden Menge an flüssiger Probe eingebracht ist. Der Anzeigestreifen 46 ragt dabei mit einem seiner Enden 47 in den Probenaufnehmer 51, wodurch ermöglicht wird, dass flüssige Probe, die vom Probenaufnehmer 51 aufgesogen wird, in den Anzeigestreifen 46 aus einem saugfähigen Material gelangen kann. Die flüssige Probe durchwandert dann den Anzeigestreifen 46 bis zu einer bestimmten Zone 48 des Anzeigestreifens 46, die in einem bestimmten Abstand vom Probenaufnehmer 51 entfernt im stäbchenförmigen Abschnitt 52 des Probenentnahmeelements 44 liegt, und über welche Zone 48 festgestellt werden kann, dass eine ausreichende Menge an flüssiger Probe im Probenaufnehmer 51 vorliegt. In dieser Zone 48 können spezifische Anzeigemittel vorgesehen sein, über welche bspw. mittels einer Farbreaktion beim Eintreffen der flüssigen Probe in der Zone 48 festgestellt werden kann, dass genügend Probe vorliegt, um den Assay durchführen zu können.

In einer weiteren Ausführungsform kann vorgesehen sein, dass der stäbchenförmige Abschnitt 52 des Probenentnahmeelements 44 eine Markierung aufweist, bspw. auf Höhe der Zone 48, die die Mindestlaufweite der flüssigen Probe auf/durch den Anzeigestreifen anzeigt. Die Markierung kann dabei bspw. ein Strich oder eine Einkerbung in dem stäbchenförmigen Abschnitt 52 sein.

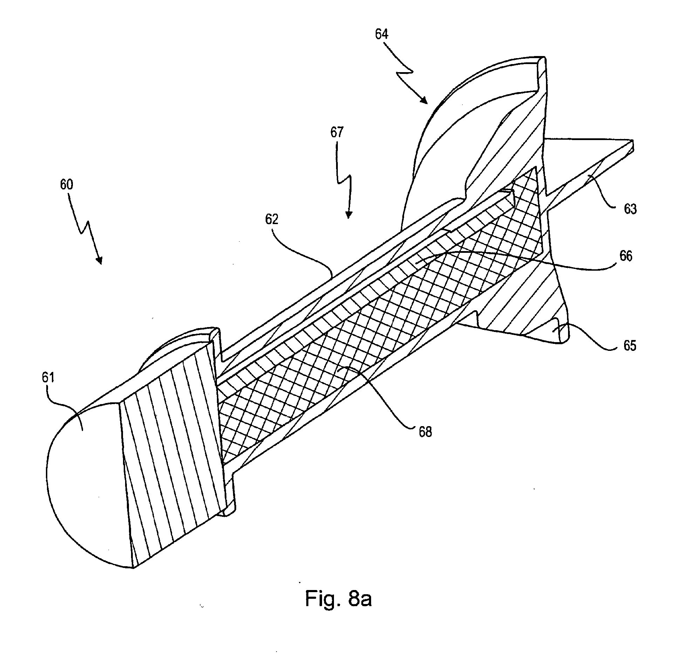

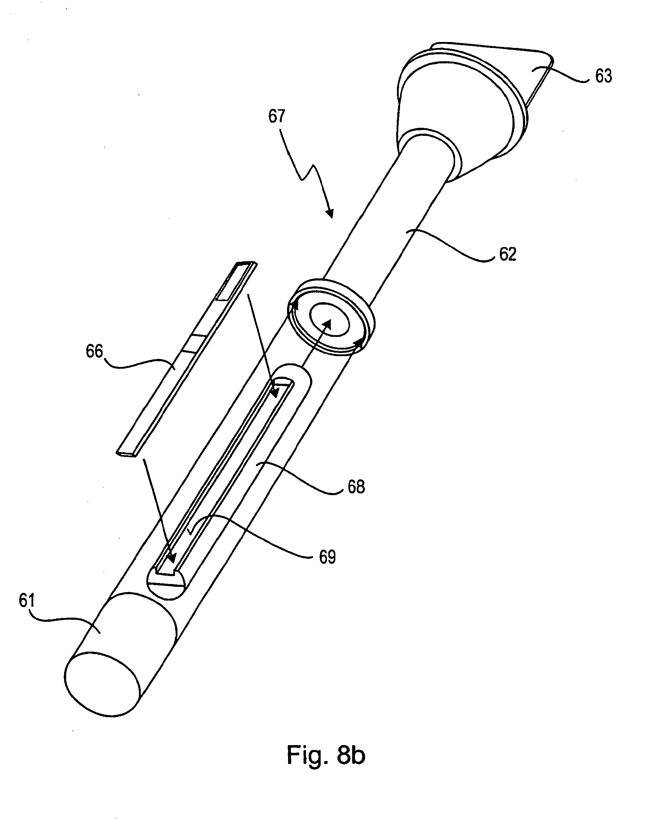

In Fig. 8 ist eine weitere Ausführungsform des Probenentnahmeelements 60 gezeigt, wobei Fig. 8A einen Längsschnitt durch das Probenentnahmeelement 60 zeigt, und Fig. 8B eine Explosionsdarstellung der einzelnen Teile bzw. Abschnitte des Probenentnahmeelements 60. Dieses weist in der in Fig. 8 gezeigten Ausführungsform ebenfalls einen Probenaufnehmer 61 auf, sowie einen länglichen, stäbchenförmigen Abschnitt 62 und einen Endabschnitt 64, der einen Griff 63 sowie Verschlussmittel 65 umfasst. Der längliche, stäbchenförmige Abschnitt 62, und der Endabschnitt 64 inklusive Griff 63 und Verschlussmittel 65 sind in der in Fig. 8 gezeigten Ausführungsform einstückig in Form eines hohlen Stäbchens 67 mit Haltegriff ausgebildet, was insbesondere in Fig. 8B zu erkennen ist. An das in das Behältnis 12 einzuführende Ende des Stäbchens 67 wird der Probenaufnehmer 61, ein schwammartiges Element, befestigt. In das Stäbchen 67 einführbar ist eine Steckstück 68, das eine - im Querschnitt gesehene - U-förmige Ausnehmung 69 aufweist, in die ein Anzeigestreifen 66 eingeführt bzw. eingelegt werden kann. Das Stäbchen 67 ist dabei aus einem transparenten Material gefertigt, so dass nach Einführen des Steckstücks 68 und dem darin liegenden Anzeigestreifens 66 in das hohle Stäbchen 67 über den Anzeigestreifen ablesbar ist, ob und wann genügend flüssige Probe über den Probenaufnehmer 61 aufgenommen ist.

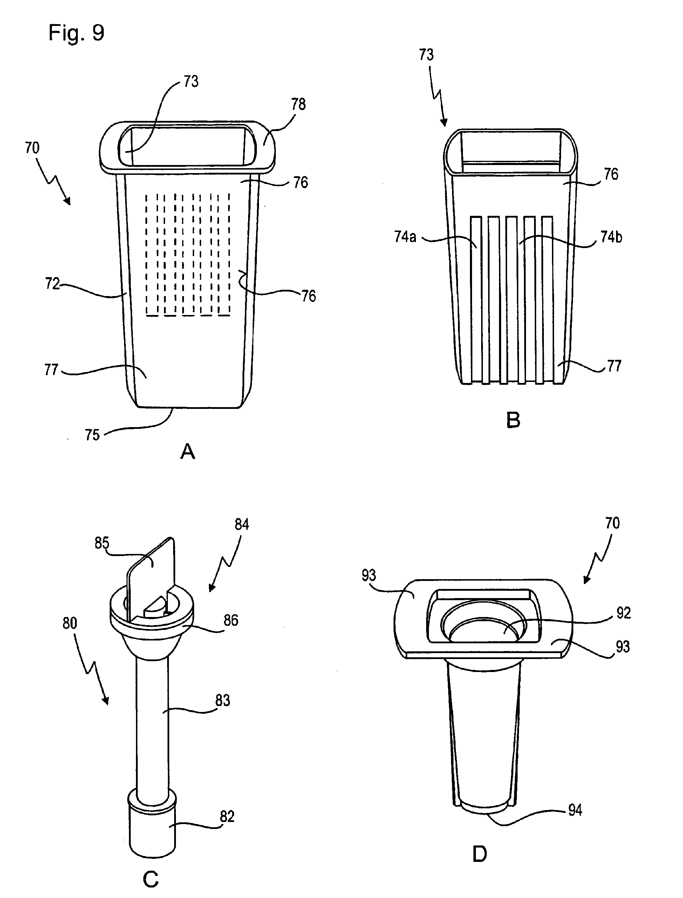

In Fig. 9 ist eine weitere Ausführungsform der erfindungsgemäßen Vorrichtung 70 bzw. dessen Teile gezeigt, mit einem Behältnis 72 und einem darin angeordneten Halteelement 73 (Fig. 9A), das in Fig. 9B auch separat dargestellt ist. Wie Fig. 9A und 9B zu entnehmen ist, weist das Halteelement 73 eine hohle eckzylindrische Form auf, die einer inneren Mantelfläche 76 des Behälters 72 angepasst ist, so dass das Halteelement 73 in das Behältnis 72 passend eingeführt werden kann. Der Boden 75 der Vorrichtung 70 ist entsprechend der eckzylindrischen Form der Mantelfläche 76 im Wesentlichen rechteckig ausgebildet. Das Halteelement 73 weist Ausnehmungen 74a auf und zwischen den Ausnehmungen 74a liegende Stege 74b, wobei beide in einem oberen, dem offenen Ende des Behältnisses 72 zugewandten Abschnitt des Halteelements 73 begrenzt sind, und zu dem Ende hin, das im eingeführten Zustand zum Boden 75 des Behältnisses 72 weist, durchgehend ausgebildet sind. In die Ausnehmungen 74a können Teststreifen eingebracht werden, die in Fig. 9 aus Gründen der Übersichtlichkeit nicht dargestellt sind. Im eingeführten Zustand endet das untere, zum Boden 75 des Behältnisses 72 weisende Ende 77 des Halteelements 73 in einem bestimmten Abstand zum Boden 75, so dass die in den Ausnehmungen 74a vorliegenden Teststreifen mit einem saugfähigen Abschnitt aus dem Halteelement 73 und den Ausnehmungen 74a derart herausragen, dass sie mit der flüssigen Probe, die vorzugsweise zwischen Boden 75 und dem unterem Ende 77 des Halteelements 73 vorliegt, in Kontakt kommen bzw. in die Probe eingetaucht werden.

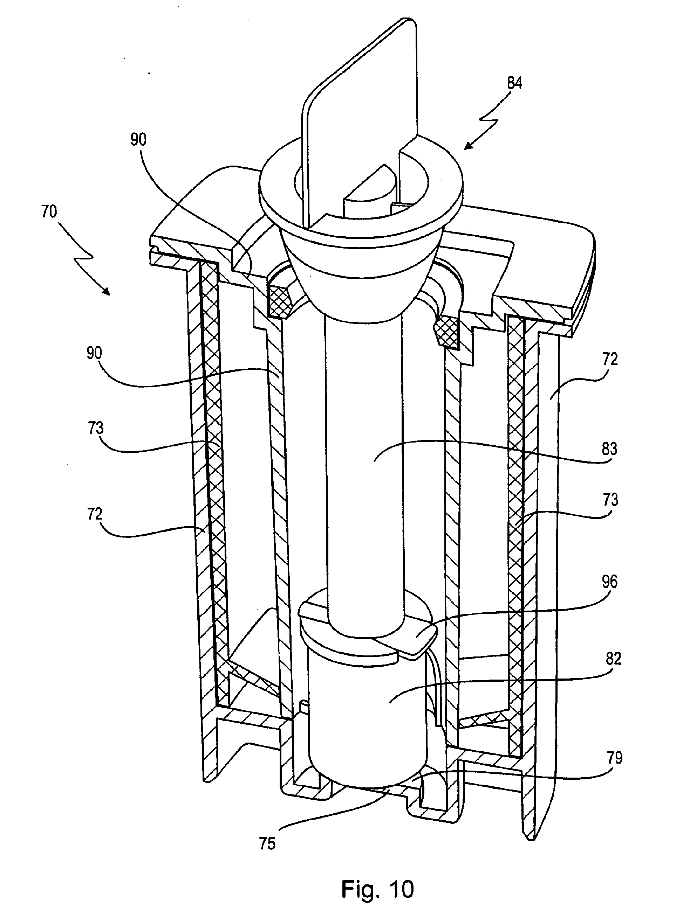

Das Ende 77 des Halteelements weist mittig ferner eine Halteelementöffnung 76 (siehe Fig. 10) auf, die derart dimensioniert ist, dass das Probenentnahmeelement 80 mit seinem Probenaufnehmer 82 durch die Halteelementöffnung 76 hindurch zur auf dem Boden 75 vorliegenden Erhebung (in Fig. 9 nicht gezeigt) geführt und auf dieser ausgepresst werden kann. Die Halteelementöffnung 76 ist dabei vorzugsweise im Wesentlichen rund bzw. zur Aufnahme und Durchführung des unteren Endes 94 des Einführstücks 90 angepasst.

In Fig. 9C ist eine Ausführungsform des Probenentnahmeelements 80 gezeigt, die bei der in Fig. 9 gezeigten Ausführungsform der Vorrichtung 70 eingesetzt werden kann, und das im Wesentlichen der in Fig. 8A und 8B gezeigten Ausführungsform des Probenentnahmeelements entspricht. Dementsprechend weist das Probenentnahmeelement 80 einen Probenaufnehmer 82 auf, ein saugfähiges, schwammähnliches Element, über das die Probe aufgenommen wird, sowie einen stäbchenförmigen Abschnitt 83, und einen Endabschnitt 84, der wiederum einen Griff 85 und Verschlussmittel 86 aufweist. Die Verschlussmittel sind in der in Fig. 9C gezeigten Ausführungsform ein umlaufender Dichtungsring, der mit einer in Fig. 9D dargestellten Öffnung 92 eines Einführstücks 90 zusammenwirkt, wenn das Probenentnahmeelement 80 in die Öffnung 92 des Einführstücks 90 eingeführt wird. Der Dichtungsring rastet dichtend in die Öffnung 92 ein, so dass ein Verrutschen des Probenentnahmeelements 80 aus dem Einführstück 90 heraus vermieden wird.

Das in Fig. 9D näher gezeigte Einführstück 90 weist eine im Wesentlichen hohle zylindrische Form auf, mit einer Öffnung 92 am Ende über das das Probenentnahmeelement 80 eingeführt wird, und mit einem offenen Ende 94, aus dem der Probenaufnehmer 82 in Richtung Boden 75 des Behältnisses 72 zumindest teilweise herausgeführt werden und dadurch auf eine auf dem Boden 75 des Behältnisses 72 vorgesehene Erhebung (in Fig. 9 nicht gezeigt) gepresst und ausgedrückt werden kann.

In Fig. 10 zeigt einen Querschnitt der zusammengesetzten Vorrichtung 70 aus Fig. 9, d.h. die Vorrichtung 70, in die das Probenentnahmeelement 80 zusammen mit dem Einführstück 90 in das Behältnis 72, in dem bereits auch das Halteelement 73 angebracht ist, eingeführt ist, bzw. gerade eingeführt wird. In Fig. 10 ist zu erkennen, dass der Boden 75 des Behältnisses 72 eine Erhebung 79 vorgesehen ist, auf die der Probenaufnehmer 82 komprimiert werden kann. Im Bereich 96 des Probenentnahmeelements 80 können weitere Mittel vorgesehen sein, die mit Fixiermitteln, bspw. einem Gewinde im Inneren bzw. an der Innenseite des Behältnisses oder des Halteelements zusammenwirken, um eine Fixierung des Probenentnahmeelements zu bewirken. Die in Fig. 10 dargestellte Erhebung 79 kann ebenfalls leistenförmige Rippen aufweisen, die aber in Fig. 10 der besseren Übersicht halber nicht dargestellt sind.

Zum Zusammensetzen der Vorrichtung 70 wird zunächst das Halteelement 73 in das Behältnis 72 eingeführt. Durch die im unteren Ende des Halteelements vorgesehene Halteelementöffnung 76 ist ein Zugang zu der Erhebung 79 am Boden 75 des Behältnisses 72 gewährleistet. Anschließend kann das Einführstück 90 eingeführt werden, das, wie in Fig. 9D zu erkennen ist, zwei seitliche Flansche bzw. einen umlaufenden Rand aufweist, der auf dem oberen Rand des Behältnisses 78 (siehe Fig. 9A) zu liegen kommt und dadurch die Einführung des Einführstücks 90 in das Behältnis 72 begrenzt. Auch das Einführstück 90, das im Wesentlichen zylinderförmig ausgebildet ist, weist am unteren, dem Boden 75 des Behältnisses 72 zugewandten Ende das offene Ende 94 auf, durch welches hindurch der Zugang zu der Erhebung 79 am Boden 75 des Behältnisses 72 für den hierauf auszudrückenden Probenaufnehmer 82 gewährleistet wird. Durch Einführen des Probenentnahmeelements 80 schließt der Endabschnitt 84 über den Dichtungsring 86 dichtend gegen die Öffnung 92 des Einführstücks 90 ab und verschließt dadurch die Vorrichtung 70. Gleichzeitig wird der Probenaufnehmer 82 auf der Erhebung 79 komprimiert, so dass die hierin zuvor aufgenommene Probe, insbesondere Speichelprobe, auf der Erhebung 79 ausgedrückt wird.

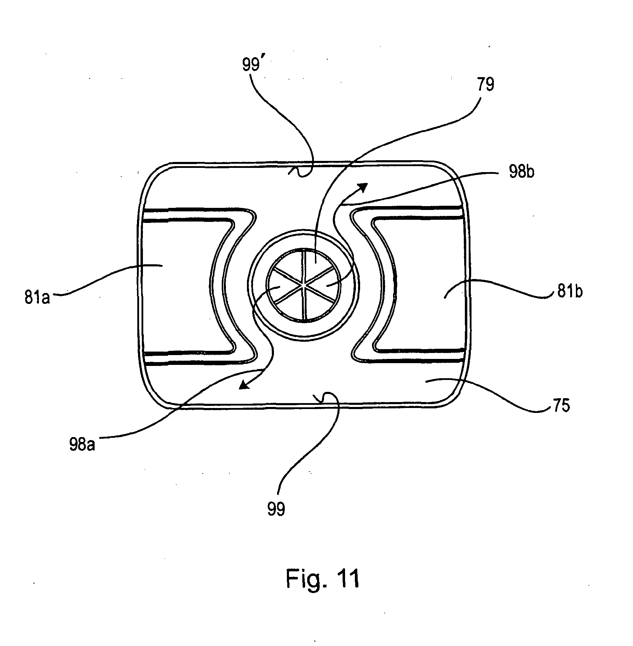

In Fig. 11 ist eine Draufsicht auf den Boden 75 des Behältnisses 72 gezeigt, wobei die umlaufende Mantelfläche 76 aus Gründen der Übersichtlichkeit nicht dargestellt ist.

Wie Fig. 11 zu entnehmen ist, befindet sich mittig auf dem Boden 75 eine halbkugelförmige Erhebung 79, auf die der Probenaufnehmer 82 ausgedrückt werden kann. Ferner befinden sich am Boden seitliche Erhebungen 81a und 81b, die in einem Abstand zur Erhebung 79 und anschleißend an die kurzen Seitenwände des im Wesentlichen rechteckigen Bodens 75 ausgebildet sind. Durch die Erhebungen 81a und 81b wird der über die Erhebung 79 ablaufende Probe bzw. der Speichel jeweils seitlich zu den langen Seitenrändern des Bodens 75 geführt, wie durch die Pfeile 98a und 98b gezeigt. In diese Seitenränder des Bodens 75 ragen von oben die saugfähigen Abschnitte der Teststreifen 18 hinein, so dass die Probe über diese saugfähigen Abschnitte auf den Teststreifen gelangen und der Test dadurch gestartet werden kann.

Es versteht sich, dass das Behältnis vorzugsweise aus einem transparenten Material, vorzugsweise Kunststoff ist, um das Testergebnis auf den Teststreifen 18 visuell oder maschinell mit einem Scanner ablesen zu können.

Es versteht sich, dass die Vorrichtung nicht auf die oben beschriebenen Ausführungsformen beschränkt ist. Insbesondere kann die Anzahl der Teststreifen beliebig variieren, und auch die Bauform des Behältnisses ist variabel und muss nicht zwingend durch ein Behältnis mit kreisförmigem Querschnitt realisiert werden. Dementsprechend kann sich auch die Form des Haltelements von derjenigen unterscheiden, welche in den Ausführungsformen beschrieben ist. Insbesondere ist es möglich, statt der gezeigten halbkugelförmigen, gewölbten Form das Halteelement rohrförmig und somit um den vollen Kreisumfang an der Innenseite der Mantelfläche anliegend auszugestalten. Ferner ist es denkbar, dass der Deckel des Behältnisses durch ein anderes Verschlusselement, insbesondere durch eine verschließbare Klappe, ersetzt wird. Auch das Abdeckelement ist nicht auf eine dreiteilige Bauform begrenzt, sondern kann auch durch ein mehrteiliges oder nur ein- oder zweiteiliges Abdeckelement realisiert werden.

Unabhängig von der jeweiligen Ausführungsform lässt sich die erfindungsgemäße Vorrichtung für jegliche Art von Drogen- bzw. Vielfachdrogentests verwenden. Dabei kann die vorliegende flüssige Probe gleichzeitig auf mehrere Missbrauchsdrogen getestet werden, darunter insbesondere Amphetamin (AMP 1000), Barbiturate (BAR 300), Benzodiazepine (BZD 300), Kokain (COC 300), Marihuana (THC 50), Methadon (MTD 300), Methamphetamin (MET 1000), Methylenedioxymethamphetamin (MDMA 500), Morphin (MOR 300), Opiate (OPI 2000), Oxycodon (OXY 100), Phencyclidin (PCP 25), Propoxyphen (PPX 300), Trizyklische, Antidepressive (TCA 1000), Buprenorphin (BUP 10), Cotinin (COT 200), EDDP (EDDP 100) und Fentanyl (FYL 10). Auch ein Alkoholtest und zumindest ein Verfälschungstest kann in die Vorrichtung integriert sein.

Die dabei leichte Handhabung der erfindungsgemäßen Vorrichtung sowie die Möglichkeit, ohne Austausch von Elementen und ohne Entfernen der Teststreifen die Probe unkontaminiert an ein zertifiziertes Labor einsenden zu können, stellen die Hauptvorteile der Erfindung dar. Dies ermöglicht eine sichere, schnelle und hygienische Durchführung des Tests und setzt keine speziellen Kenntnisse des Prüfers voraus.

## Patentansprüche

1. Vorrichtung (10; 70) zum Bestimmen des Vorliegens und/oder der Menge eines oder mehrerer Analyten in einer Probe einer Körperflüssigkeit eines Menschen, aufweisend:
- ein Behältnis (12; 72) zum Aufnehmen einer Körperflüssigkeitsprobe, mit einem Innenraum, der von einem Boden (15; 75) und einer Mantelfläche (16; 76) begrenzt wird,
- zumindest einen Teststreifen (18) mit einem saugfähigen Abschnitt (19) sowie mit Reagenzien zum Bestimmen des Vorliegens und/oder der Menge eines oder mehrerer Analyten in der Körperflüssigkeitsprobe, und
- ein Halteelement (13; 73) zur Aufnahme und zum Halten des oder der Teststreifen (18),
wobei die Vorrichtung ferner ein längliches Probenentnahmeelement (20; 44; 60; 80) aufweist, das an einem seiner Enden einen saugfähigen, die Körperflüssigkeitsprobe aufnehmenden Probenaufnehmer (22; 51; 61; 82) aufweist, welches Probenentnahmeelement (20; 44; 60; 80) in das Behältnis (12; 72) zur Überführung der Körperflüssigkeitsprobe in das Behältnis (12; 72) über sein den Probenaufnehmer (22; 51; 61; 82) aufweisendes Ende einführbar ist, und dass der Boden (15; 75) des Behältnisses (12; 72) eine zentrale Erhebung (30; 79) aufweist, über welche die Körperflüssigkeitsprobe vom Probenaufnehmer (22; 51; 61; 82) durch dessen Komprimieren auf der Erhebung (30; 79) dem zumindest einen Teststreifen (18) zuführbar ist, **dadurch gekennzeichnet, dass** das Halteelement (13; 73) einer der umlaufenden Mantelfläche (16; 76) des Behältnisses (12; 72) entsprechende und angepasste Form aufweist, und dass
das Probenentnahmeelement (20; 44; 60; 80) und/oder der Probenaufnehmer (22; 51; 61; 82) Mittel (45; 66) zum Anzeigen des Vorliegens einer Menge der flüssigen Probe, die zum Bestimmen des Vorliegens und/oder der Menge eines oder mehrerer Analyten in der Probe ausreichend ist, aufweist.

2. Vorrichtung (10; 70) nach Anspruch 1, **dadurch gekennzeichnet, dass** die Erhebung (16; 79) eine halbkugelförmige Erhebung (30; 79) mittig im Boden (15; 75) des Behältnisses (12; 72) darstellt.

3. Vorrichtung (10; 70) nach Anspruch 1 oder 2, **dadurch gekennzeichnet, dass** die Erhebung (30; 79) leistenförmige Elemente (32) aufweist, die mittig von der Erhebung (30; 79) zum Boden (15; 75) hin geführt sind zum Ableiten der Körperflüssigkeitsprobe an den umlaufenden Rand des Bodens (15; 75) des Behältnisses (12; 72).

4. Vorrichtung (10; 70) nach einem der Ansprüche 1 bis 3, **dadurch gekennzeichnet, dass** der Boden (15; 75) des Behältnisses (12; 72) im Bereich und umlaufend an der Mantelfläche entlang eine Vertiefung (34) aufweist, zur Aufnahme der von der Erhebung (30; 79) abfließenden Körperflüssigkeitsprobe.

5. Vorrichtung (10; 70) nach einem der Ansprüche 1 bis 4, **dadurch gekennzeichnet, dass** das Probenentnahmeelement (20; 44; 60; 80) an seinem dem Ende mit dem Probenaufnehmer (22; 51; 61; 82) entgegen gesetzten Ende (24) ein Verschlussmittel (26; 65; 86) zum Verschließen des Behältnisses (12; 72) nach Einbringen des Probenentnahmeelements (20; 44; 60; 80) in das Behältnis (12; 72) aufweist.

6. Vorrichtung (10; 70) nach Anspruch 5, **dadurch gekennzeichnet, dass** an dem Verschlussmittel (26; 65; 86) zumindest ein Rastmittel vorgesehen ist, über welches das Herausdrehen oder -ziehen des Probenentnahmeelements (20; 60; 80) nach dessen Einbringung in das Behältnis (12; 72) verhindert wird.

7. Vorrichtung (10; 70) nach einem der Ansprüche 5 bis 6, **dadurch gekennzeichnet, dass** das Verschlussmittel (26) ein durchstechbares, selbstabdichtendes Probeentnahmemittel (40) aufweist, über welches das Innere des Behältnisses (12; 72) nach dessen Durchstechen auch nach Aufbringung des Verschlussmittels (26) auf das Behältnis (12; 72) zugänglich ist.

8. Vorrichtung (10; 70) nach Anspruch einem der vorstehenden Ansprüche, **dadurch gekennzeichnet, dass** die Mittel (45) ein in dem Probenentnahmeelement (20; 44; 60; 80) vorliegender Anzeigestreifen (46; 66) aus einem saugfähigen Material sind.

9. Vorrichtung (10; 70) nach Anspruch 8, **dadurch gekennzeichnet, dass** der Anzeigestreifen (46; 66) eine Zone (48) zum Anzeigen des Vorliegens der ausreichenden Menge aufweist.

10. Vorrichtung (10; 70) nach Anspruch 8 oder 9, **dadurch gekennzeichnet, dass** das Probenentnahmeelement (20; 44; 60; 80) eine Markierung aufweist, die die Mindestlaufweite der Körperflüssigkeitsprobe durch den Anzeigestreifen (46; 66) anzeigt.

11. Vorrichtung (10; 70) nach einem der vorstehenden Ansprüche, **dadurch gekennzeichnet, dass** das Haltelement (13; 73) vorgespannt geformt und in das Behältnis (12; 72) geklemmt angeordnet ist.

12. Vorrichtung (10; 70) nach einem der vorstehenden Ansprüche, **dadurch gekennzeichnet, dass** zwischen 1 und 12 Teststreifen (18) vorgesehen sind.

13. Vorrichtung (10; 70) nach einem der vorstehenden Ansprüche, **dadurch gekennzeichnet, dass** Teststreifen zum Nachweis von zumindest einer Droge und/oder Arzneimittels vorgesehen ist, die/das ausgewählt ist aus Amphetamin, Barbiturate, Benzodiazepine, Kokain, Marihuana, Methadon, Methamphetamin, Methylenedioxymethamphetamin, Morphin, Opiate, Oxycodon, Phencyclidin, Propoxyphen, Trizyklische Antidepressiva, Buprenorphin, Cotinin, EDDP (2-Ethylidin-1,5-Dimethyl-3,3-Diphenylpyrrolidin), Fentanyl, Alkohol.

14. Vorrichtung (10; 70) nach einem der vorstehenden Ansprüche, **dadurch gekennzeichnet, dass** das Halteelement (13; 73) im Vergleich zu dem zumindest einen Teststreifen (18) eine dunklere Einfärbung aufweist.

15. Vorrichtung (10; 70) nach einem der vorstehenden Ansprüche, **dadurch gekennzeichnet, dass** das Probenentnahmeelement (20; 44; 60; 80) ein hohles, stäbchenförmiges Element 67 ist, mit an seinem in das Behältnis (12; 72) einzuführende Ende angebrachten Probenaufnehmer (22; 51; 61; 82) und mit einem in dem stäbchenförmigen Element (67) angeordnetem Steckstück 68, das eine Ausnehmung (69) für ein Anzeigestreifen (66) aufweist.

## Claims

1. A device (10; 70) for determining the presence and/or the quantity of one or more analytes in a sample of human body fluid, wherein the device comprises:
- a container (12; 72) for receiving a sample of body fluid, with an interior that is delimited by a base (15; 75) and a circumferential surface (16; 76);
- at least one test strip (18) including an absorbent section (19) and reagents for determining a presence and/or a quantity of one or more analytes in the sample of body fluid, and
- a holding element (13; 73) for receiving and holding the one or more test strip (18), wherein the device further comprises an elongate sampling element (20; 44; 60; 80) which includes, at one of its ends, an absorbent receiving sampler (20; 51; 61; 62) that takes up the body fluid,
wherein the sampling element (20; 44; 60; 80) is insertable into the container (12; 72) for transfer of the sample of body fluid into the container (12; 72) by transferring the sample of body fluid into the container (12; 72) via the end comprising the sampler (22; 51; 61; 62), and wherein the base (15; 72) of the container (12; 72) comprises a central elevation (30; 79) by means of which the sample of body fluid is prondable from the sampler (22; 51; 61; 82) to the at least one test strip (16) by compressing the sampler on the elevation (30; 79), **characterized in that** the holding element has a shape corresponding and adapted to the peripheral circumferential surface (16; 76) of the container (12; 72) and **in that** the sampling element (20; 44; 60; 80) and/or the sampler (22; 51; 61; 82) includes means (45; 66) for indication of the presence of a quantity of the liquid sample sufficient for determining the presence and/or the quantity of one or more analytes in the sample.

2. The device (10; 70) of claim 1, **characterized in that** the elevation (16; 79) is a hemispherical elevation (30; 79) arranged centrally on the base (15; 75) or the container (12; 72).

3. The device of claim 1 or 2, **characterized in that** the elevation (30; 79) comprises ridge-shaped elements (32) which extend centrally from the elevation (30; 79) towards the base (15; 75) for conducting the sample of body fluid to the peripheral edge of the base (15; 75) of the container (12; 72).

4. The device (10; 70) of any of claims 1 through 3, **characterized in that** the base (15; 75) of the container (12; 72) comprises a depression (34) in the area of and extending around the circumferential surface for receiving the sample of body fluid flowing down of the elevation (30; 79).

5. The device (10; 70) of any of claims 1 through 4, **characterized in that** the sampling element (20; 44; 60; 80), at its end remote from the end with the sampler (22; 51; 61; 81), has a closure means (26; 65, 86) for closing the container (12; 72) after the sampling element (20; 44; 60; 80) has been introduced into the container (12; 72).

6. The device (10; 70) of claim 5, **characterized in that** the closure means (26; 65; 86) is providing with at least one blocking means for blocking the sampling element (20; 60; 80) being screwed out or pulled out after having been introduced into the container (12; 72).

7. The device (10; 70) of any of claims 5 through 6, **characterized in that** the closure means (26) comprises a pierceable self-sealing sampling means (40) for permitting, after it has been pierced, access to the interior of the container (12; 72) even after the closure means (26) has been fitted to the container (12; 72).

8. The device (10; 70) of any of the above claims, **characterized in that** the means (45) are an indicator strip (46; 66) of absorbent material present in the sampling element (20; 44; 66; 80).

9. The device (10; 70) of claim 8, **characterized in that** the indicator strip (46; 66) comprises a zone (48) for indicating the presence of the sufficient quantity.

10. The device (10; 70) of claim 8 or 9, **characterized in that** the sampling element (20; 44; 60; 80) comprises a marking indicating the minimum traveling distance of a sample of body fluid through the indicators (46; 66).

11. The device (10; 70) of any of the above claims, **characterized in that** the holding element (13; 73) is shaped for having a pretension and is located clamped into the container (12; 72).

12. The device (10; 70) of any of the above claims, **characterized in that** between 1 and 12 test strips (18) are provided.

13. The device (10; 70) of any of the above claims, **characterized in that** test strips are provided for the detection of at least one drug and/or medicament selected from among amphetamines, barbiturates, benzodiazepines, cocaine, marihuana, methadone, methamphetamine, methylenedioxy-methamphetamine, morphine, opiates, oxycodone, phencyclidine, propoxyphene, tricyclic antidepressants, buprenorphine, cotinine, EDDP (2-ethylidene-1,5-dimethyl-3,3-diphenylpyrrolidine), fentanyl, alcohol.

14. The device (10; 70) of any of the above claims, **characterized in that** the holding element (13; 73) comprises a darker coloring in comparison to the test strip (18).

15. The device (10; 70) of any of the above claims, **characterized in that** the sampling element (20; 44; 60; 80) is hollow, rod-shaped element (67), with the sampler (22; 51; 61; 82) arranged on one of its ends to be inserted into the container (12; 72) and with an insertion element (68) arranged in the rod-shaped element (67) and having a recess (69) for the indicator strip (66).

## Revendications

1. Dispositif (10 ; 70) permettant de déterminer la présence et/ou la quantité d'un ou de plusieurs analytes dans un échantillon d'un liquide organique d'un homme, comportant :
- un récipient (12 ; 72) destiné à recevoir un échantillon de liquide organique et comportant une enceinte intérieure, qui est délimitée par un fond (15 ; 75) et une paroi latérale (16 ; 76),
- au moins une bandelette réactive (18) avec une partie absorbante (19) et avec des réactifs permettant de déterminer la présence et/ou la quantité d'un ou de plusieurs analytes dans l'échantillon de liquide organique, et
- un élément de fixation (13 ; 73) destiné à recevoir et à maintenir la ou les bandelettes réactives (18),
ledit dispositif comportant, en outre, un élément de prélèvement d'échantillon (20 ; 44 ; 60 ; 80) allongé, qui comporte, au niveau de l'une de ses extrémités, un récepteur d'échantillon (22 ; 51 ; 61 ; 82) absorbant, destiné à prélever l'échantillon de liquide organique, lequel élément de prélèvement d'échantillon (20 ; 44 ; 60 ; 80) est propre à être introduit dans le récipient (12 ; 72) pour transférer l'échantillon de liquide organique dans le récipient (12 ; 72) par l'intermédiaire de son extrémité munie du récepteur d'échantillon (22 ; 51 ; 61 ; 82), et le fond (15 ; 75) du récipient (12 ; 72) comportant un renflement (30 ; 79) central, par l'intermédiaire duquel l'échantillon de liquide organique peut être amené vers ladite au moins une bandelette réactive (18) par le récepteur d'échantillon (22 ; 51 ; 61 ; 82) sous l'effet de la compression de celui-ci sur le renflement (30 ; 79),
**caractérisé en ce que** l'élément de prélèvement d'échantillon (20 ; 44 ; 60 ; 80) et/ou le récepteur d'échantillon (22 ; 51 ; 61 ; 82) comportent des moyens (45 ; 66) destinés à signaler la présence d'une quantité d'échantillon liquide qui est suffisante pour déterminer la présence et/ou la quantité d'un ou de plusieurs analytes dans l'échantillon,
et **en ce que** l'élément de fixation (13 ; 73) possède une forme ajustée et conforme à la paroi latérale (16 ; 76) périphérique du récipient (12 ; 72).

2. Dispositif (10 ; 70) selon la revendication 1, **caractérisé en ce que** le renflement (16 ; 79) constitue un renflement (30 ; 79) semi-sphérique au milieu du fond (15 ; 75) du récipient (12 ; 72).

3. Dispositif (10 ; 70) selon la revendication 1 ou 2, **caractérisé en ce que** le renflement (30 ; 79) comporte des éléments (32) en forme de moulures qui sont guidées depuis le milieu du renflement (30 ; 79) vers le fond (15 ; 75) afin de faire dériver l'échantillon de liquide organique sur le bord périphérique du fond (15 ; 75) du récipient (12 ; 72).

4. Dispositif (10 ; 70) selon l'une quelconque des revendications 1 à 3, **caractérisé en ce que** le fond (15 ; 75) du récipient (12 ; 72) comporte, dans la zone de la paroi latérale et tout le long de celle-ci, un creux (34) destiné à recevoir l'échantillon de liquide organique qui s'écoule depuis le renflement (30 ; 79).

5. Dispositif (10 ; 70) selon l'une quelconque des revendications 1 à 4, **caractérisé en ce que** l'élément de prélèvement d'échantillon (20 ; 44 ; 60 ; 80), au niveau de son extrémité (24) opposée à l'extrémité munie du récepteur d'échantillon (22 ; 51 ; 61 ; 82), comporte un moyen de fermeture (26 ; 65 ; 86) permettant de fermer le récipient (12 ; 72) après l'introduction de l'élément de prélèvement d'échantillon (20 ; 44 ; 60 ; 80) dans le récipient (12 ; 72).

6. Dispositif (10 ; 70) selon la revendication 5, **caractérisé en ce que** sur le moyen de fermeture (26 ; 65 ; 86) est prévu au moins un moyen de blocage, qui empêche que l'élément de prélèvement d'échantillon (20 ; 60 ; 80) après son introduction dans le récipient (12 ; 72) puisse être extrait de celui-ci par vissage ou traction.

7. Dispositif (10 ; 70) selon l'une quelconque des revendications 5 à 6, **caractérisé en ce que** le moyen de fermeture (26) comporte un moyen de prélèvement d'échantillon (40) auto-étanche apte à être transpercé, qui, après son transpercement, permet d'accéder à l'intérieur du récipient (12 ; 72), même après la pose du moyen de fermeture (26) sur le récipient (12 ; 72).

8. Dispositif (10 ; 70) selon l'une quelconque des revendications précédentes, **caractérisé en ce que** les moyens (45) sont une bandelette d'affichage (46 ; 66) en matériau absorbant, disposée dans l'élément de prélèvement d'échantillon (20 ; 44 ; 60 ; 80).

9. Dispositif (10 ; 70) selon la revendication 8, **caractérisé en ce que** la bandelette d'affichage (46 ; 66) comporte une zone (48) destinée à signaler la présence de la quantité suffisante.

10. Dispositif (10 ; 70) selon la revendication 8 ou 9, **caractérisé en ce que** l'élément de prélèvement d'échantillon (20 ; 44 ; 60 ; 80) comporte un repère qui affiche la propagation minimum de l'échantillon de liquide organique à travers la bandelette d'affichage (46 ; 66).

11. Dispositif (10 ; 70) selon l'une quelconque des revendications précédentes, **caractérisé en ce que** l'élément de fixation (13 ; 73) est formé avec une précontrainte et est disposé en étant serré dans le récipient (12 ; 72).

12. Dispositif (10 ; 70) selon l'une quelconque des revendications précédentes, **caractérisé en ce qu'**il est prévu entre 1 et 12 bandelettes réactives (18).

13. Dispositif (10 ; 70) selon l'une quelconque des revendications précédentes, **caractérisé en ce qu'**il est prévu des bandelettes réactives permettant de déceler au moins une drogue et/ou un produit pharmaceutique choisie/choisi parmi les amphétamines, les barbituriques, les benzodiazépines, la cocaïne, la marijuana, la méthadone, les méthamphétamines, les méthylènedioxyméthamphétamines, la morphine, les opiacés, l'oxycodone, la phéncyclidine, le propoxyphène, les antidépresseurs tricycliques, la buprénorphine, la cotinine, l'EDDP (2-éthylidine-1,5-diméthyl-3,3-diphénylpyrrolidine), le fentanyl, l'alcool.

14. Dispositif (10 ; 70) selon l'une quelconque des revendications précédentes, **caractérisé en ce que** l'élément de fixation (13 ; 73) possède une coloration plus sombre que ladite au moins une bandelette réactive (18).

15. Dispositif (10 ; 70) selon l'une quelconque des revendications précédentes, **caractérisé en ce que** l'élément de prélèvement d'échantillon (20 ; 44 ; 60 ; 80) est un élément (67) creux en forme de bâtonnet, avec un récepteur d'échantillon (22 ; 51 ; 61 ; 82) agencé à son extrémité destinée à être introduite dans le récipient (12 ; 72), et avec une partie enfichable (68) agencée sur l'élément (67) en forme de bâtonnet et comportant un évidement (69) pour une bandelette d'affichage (66).
